# EUROPEAN PATENT SPECIFICATION

(11) **EP 2 370 179 B1**
(45) Date of publication and mention of the grant of the patent: **23.03.2016**
(21) Application number: 09748781.3
(22) Date of filing: 06.11.2009
(51) Int. Cl.: A61Q 17/04, A61K 8/02, A61K 8/04, A61K 8/06, A61K 8/29, A61K 8/35, A61K 8/40, A61K 8/46, A61K 8/49, A61K 8/73, A61K 8/88

(54) **PHOTOPROTECTIVE COMPOSITION CONTAINING AN UNMODIFIED GELLING STARCH AND POLYAMIDE PARTICLES**
LICHTSCHUTZZUSAMMENSETZUNG MIT EINER UNMODIFIZIERTEN GELIERENDEN STÄRKE UND POLYAMID-TEILCHEN
COMPOSITION PHOTOPROTECTRICE CONTENANT UN AMIDON GÉLIFIANT NON MODIFIÉ ET DES PARTICULES DE POLYAMIDE

(30) Priority: 25.11.2008 FR 0858003; 02.12.2008 US 193461 P
(43) Date of publication of application: 05.10.2011
(73) Proprietor: L'Oréal, 75008 Paris (FR)
(72) Inventor: CHEVALIER, Cyril, F-91260 Juvisy-sur-Orge (FR); DUFFET, Vanessa, F-94340 Joinville-le-pont (FR); GUIRAMAND, Carole, F-78350 Jouy En Josas (FR)
(74) Representative: Miszputen, Laurent
(86) International application number: PCT/EP2009/064760
(87) International publication number: WO 2010/060776

(56) References cited:
- EP-A- 1 319 395
- EP-A- 1 371 358
- EP-A- 1 604 644
- WO-A-2004/045570
- WO-A-2008/018028

## Description

The present invention relates to a composition intended for protecting the skin and/or hair against ultraviolet radiation, characterized by the fact that it comprises, in a cosmetically acceptable support containing one aqueous phase, at least:
(a) a photoprotective system capable of screening out UV radiation;
(b) at least one gelling starch that is not modified by a chemical or physical process **which is chosen from unmodified maize starches;** and
(c) polyamide particles.

It is known that light radiation with wavelengths between 280 nm and 400 nm permits tanning of the human epidermis and that rays with wavelengths between 280 and 320 nm, which are known as UV-B rays, cause skin burns and erythema that can harm the development of a natural tan; this UV-B radiation should thus be screened out.

It is also known that UV-A rays, with wavelengths between 320 and 400 nm, which cause tanning of the skin, are liable to induce impairment thereof, especially in the case of sensitive skin or of skin that is continually exposed to solar radiation. UV-A rays in particular cause a loss of skin elasticity and the appearance of wrinkles, leading to premature ageing. They promote the onset of the erythema reaction or amplify this reaction in the case of certain individuals, and may even be the cause of phototoxic or photoallergic reactions. It is thus desirable also to screen out UV-A radiation.

Many photoprotective (UV-A and/or UV-B) cosmetic compositions for the skin have been proposed to date. Both fluid formulations that afford for the users easy application to the skin and more viscous formulations for smaller areas of the skin are desired.

These photoprotective compositions are usually in the form of an emulsion of oil-in-water type (that is to say a cosmetically acceptable support constituted of an aqueous dispersing continuous phase and of an oily dispersed discontinuous phase) or of water-in-oil type (that is to say a cosmetically acceptable support constituted of an oily dispersing continuous phase and of an aqueous dispersed discontinuous phase), which contains, in varying concentrations, one or more conventional lipophilic and/or hydrophilic organic screening agents capable of selectively absorbing the harmful UV rays. They are used for products with daily applications and especially for anti-sun products used in beach/sea or mountain conditions.

These anti-sun products should, on the one hand, have a good physicochemical stability over time and to temperature variations. On the other hand, they should provide a good UV protection and contain sufficient UV-screening agent in a harmonious manner in order to obtain the desired sun protection factor (SPF) values that are expressed mathematically by the ratio of the dose of UV radiation needed to reach the erythemogenic threshold with the UV-screening agent to the dose of UV radiation needed to reach the erythemogenic threshold without UV-screening agent, and also the desired values of the UV-A protection index according to the PPD (Persistent Pigment Darkening) method which is expressed mathematically by the ratio of the dose of UV-A radiation needed to reach the pigmentation threshold with the UV-screening agent (MPPDp) to the dose of UV-A radiation needed to reach the pigmentation threshold without UV-screening agent (MPPDₙₚ).

However, the incorporation of large amounts of organic UV-screening agents in order to obtain the desired UV protection usually leads to an impairment of the sensory and visual cosmetic properties on the skin after application which is expressed by a tacky effect, a greasy effect, a shiny effect and/or the presence of a residual film which are unpleasant for the comfort of the user.

Therefore, there is a growing need for anti-sun products that combine good performances as regards the SPF and PPD indices, good physicochemical stability over time and with respect to temperature and good cosmetic properties after application without a tacky effect, without a greasy effect, without a shiny effect and without the presence of a residual film ("bare skin" effect).

It is known from patent applications EP 925 777 and EP 745 379 to use pure starches of food origin or certain starches that are modified as regards the amylose/amylopectin ratio or that are modified by crosslinking as fillers in cosmetic compositions. Starches are also known, in application EP 1 230 914, in cosmetics as anti-pollution agents.

WO2004045570 discloses in example 4 an emulsion comprising UV filters, tapioca starch and polyamide-6. The examples 1 and 7 of EP1371358 disclose O/W-emulsions comprising UV filters, a polyamide (nylon-12) and a modified starch (diphosphate starch).

However, after considerable research conducted in the field of photoprotection mentioned above, the Applicant has now discovered, surprisingly, that the use of the combination of a maize gelling starch that is not modified by a chemical or physical process and of polyamide particles in an aqueous composition containing at least one system for screening out UV radiation makes it possible to obtain anti-sun products that combine good performances as regards the SPF and PPD indices, good physicochemical stability over time and with respect to temperature and also good cosmetic properties after application without a tacky effect, without a greasy effect, without a shiny effect and without the presence of a residual film ("bare skin" effect).

This discovery forms the basis of the present invention.

Thus, in accordance with a first subject of the present invention, a composition intended for protecting the skin and/or hair against ultraviolet radiation is proposed, characterized by the fact that it comprises, in a cosmetically acceptable support containing at least one aqueous phase, at least:
(a) a photoprotective system capable of screening out UV radiation;
(b) at least one gelling starch that is not modified by a chemical or physical process **which is chosen from unmodified maize starches;** and
(c) polyamide particles.

According to the invention, the term "photoprotective system capable of screening out UV radiation" is generally intended to denote any compound or any combination of compounds which, via mechanisms known per se of absorption and/or reflection and/or scattering of UV-A and/or UV-B radiation, makes it possible to prevent, or at least to limit, the contact of the said radiation with a surface (skin or hair) onto which this or these compound(s) have been applied. In other words, these compounds may be UV-absorbing photoprotective organic screening agents or UV-scattering and/or UV-reflecting mineral pigments, and also mixtures thereof.

The term "cosmetically acceptable" means compatible with the skin and/or its integuments, which has a pleasant colour, odour and feel and which does not cause any unacceptable discomfort (stinging, tautness or redness) liable to put the consumer off using this composition.

The expression "gelling starch" is understood to mean any starch which dissolves in water and which thickens and/or gels the composition, either at ambient temperature (75°C) or under the action of a temperature of at least 70°C for at least half an hour.

Yet another subject of the present invention lies in the use of the combination of at least one gelling starch **which is chosen from unmodified maize starches** and of polyamide particles in a composition comprising, in a support that comprises at least one aqueous phase and that is cosmetically acceptable, at least one photoprotective system capable of screening out UV radiation, for the purpose of improving the cosmetic properties after application, **said properties being chosen from** the non-tacky effect, non-greasy effect, non-shiny effect and/or the absence of residual film.

Other characteristics, aspects and advantages of the present invention will emerge on reading the detailed description that follows.

The gelling starches that can be used in the present invention are more particularly macromolecules in the form of polymers composed of elementary units which are anhydroglucose units. The number of these units and the assembly thereof make it possible to distinguish amylose (linear polymer) and amylopectin (branched polymer). The relative proportions of amylose and of amylopectin, and also the degree of polymerization thereof, varies as a function of the botanical origin of the starches. The amylose/amylopectin weight ratio may vary from 30/70 (maize) to 16/84 (rice). The weight-average molecular weight of the amylose is preferably up to 1 million and that of the amylopectin is preferably from 100 to 500 million.

The starch molecules used in the present invention are made of unmodified maize starches (INCI name: ZEA MAYS STARCH) such as the products sold under the trade names Farmal CS, in particular the commercial product Farmal CS 3650 from Corn Products International, or modified maize starches.

According to the invention, the unmodified gelling starch may preferably represent from 0.1 to 10% by weight, preferably from 0.5 to 5% by weight and more particularly from 1 to 3% by weight relative to the total weight of the final composition.

As a general rule, the polyamide particles used according to the invention are listed under the CTFA name of "nylon 12" or "nylon 6".

The polyamide particles used in the invention may be those sold under the name ORGASOL by ATOCHEM. The process for obtaining these particles is, for example, that described in document FR 2 619 385 or in document EP 303 530. These polyamide particles are furthermore known according to their various physicochemical properties under the name of "polyamide PA-12" or "polyamide PA-6".

The particles used in the invention may also be those sold under the name SP500 by KOBO.

In the compositions according to the invention, the particles have a density ranging from 1 g/cm³ to 1.84 g/cm³ and in particular a density ranging from 1.0 g/cm³ to 1.4 g/cm³.

The polyamide particles of the invention are generally spherical and solid; they especially have average dimensions ranging from 5 µm to 50 µm and more particularly ranging from 10 µm to 30 µm.

The polyamide particles are present in the compositions of the invention in concentrations preferably ranging from 0.1% to 10% by weight and more particularly from 0.5% to 5% by weight relative to the total weight of the composition.

According to the invention, the photoprotective system may be constituted of one or more hydrophilic, lipophilic or insoluble organic screening agents and/or one or more inorganic screening agents. Preferentially, it will be constituted of at least one hydrophilic, lipophilic or insoluble organic UV-screening agent in the customary cosmetic solvents.

The organic UV-screening agents are chosen especially from cinnamic derivatives; anthranilates; salicylic derivatives; dibenzoylmethane derivatives; camphor derivatives; benzophenone derivatives; **β,β**-diphenyl-acrylate derivatives; triazine derivatives; benzotriazole derivatives; benzoalmalonate derivatives, especially those cited in patent US 5 624 663; benzimidazole derivatives; imidazolines; bis-benzoazolyl derivatives as described in patents EP 669 323 and US 2 463 264; p-aminobenzoic acid (PABA) derivatives; methylenebis(hydroxyphenylbenzotriazole) derivatives as described in patent applications US 5 237 071, US 5 166 355, GB 2 303 549, DE 197 26 184 and EP 893 119; benzoxazole derivatives as described in patent applications EP 0 832 642, EP 1 027 883, EP 1 300 137 and DE 101 62 844; screening polymers and screening silicones such as those described especially in patent application WO 93/04665; α,-alkylstyrene-derived dimers such as those described in patent application DE 198 55 649; 4,4-diarylbutadienes such as those described in patent applications EP 0 967 200, DE 197 46 654, DE 197 55 649, EP-A-1 008 586, EP 1 133 980 and EP 133 981, merocyanin derivatives such as those described in patent applications WO 04/006878, WO 05/058269 and WO 06/032741; and mixtures thereof.

As examples of complementary organic photoprotective agents, mention may be made of those denoted hereinbelow under their INCI name:

### Cinnamic derivatives:

Ethylhexyl methoxycinnamate sold in particular under the trade name "Parsol MCX" by Hoffmann LaRoche,
Isopropyl methoxycinnamate,
Isoamyl methoxycinnamate sold under the trade name "Neo Heliopan E 1000" by Haarmann and Reimer,
DEA methoxycinnamate,
Diisopropyl methylcinnamate,
Glyceryl ethylhexanoate dimethoxycinnamate.

### Dibenzoylmethane derivatives:

Butylmethoxydibenzoylmethane sold especially under the trade name "Parsol 1789" by Hoffmann LaRoche, Isopropyldibenzoylmethane.

### para-Aminobenzoic acid derivatives:

PABA,
Ethyl PABA,
Ethyl dihydroxypropyl PABA,
Ethylhexyl dimethyl PABA sold in particular under the name "Escalol 507" by ISP,
Glyceryl PABA,
PEG-25 PABA sold under the name "Uvinul P25" by BASF.

### Salicylic derivatives:

Homosalate sold under the name "Eusolex HMS" by Rona/EM Industries,
Ethylhexyl salicylate sold under the name "Neo Heliopan OS" by Haarmann and Reimer,
Dipropylene glycol salicylate sold under the name "Dipsal" by Scher,
TEA salicylate sold under the name "Neo Heliopan TS" by Haarmann and Reimer.

### β,β-Diphenylacrylate derivatives:

Octocrylene sold in particular under the trade name "Uvinul N539" by BASF,
Etocrylene sold in particular under the trade name "Uvinul N35" by BASF.

### Benzophenone derivatives:

Benzophenone-1 sold under the trade name "Uvinul 400" by BASF,
Benzophenone-2 sold under the trade name "Uvinul D50" by BASF,
Benzophenone-3 or Oxybenzone sold under the trade name "Uvinul M40" by BASF,
Benzophenone-4 sold under the trade name "Uvinul MS40" by BASF,
Benzophenone-5,
Benzophenone-6 sold under the trade name "Helisorb 11" by Norquay,
Benzophenone-8 sold under the trade name "Spectra-Sorb UV-24" by American Cyanamid,
Benzophenone-9 sold under the trade name "Uvinul DS-49" by BASF,
Benzophenone-12,
n-Hexyl 2-(4-diethylamino-2-hydroxybenzoyl)benzoate sold under the trade name "Uvinal A+" or as a mixture with octylmethoxycinnamate under the trade name "Uvinul A+B" by BASF.

### Benzylidenecamphor derivatives:

3-Benzylidenecamphor manufactured under the name "Mexoryl SD" by Chimex,
4-Methylbenzylidenecamphor sold under the name "Eusolex 6300" by Merck,
Benzylidenecamphor sulphonic acid manufactured under the name "Mexoryl SL" by Chimex,
Camphor benzalkonium metho sulphate manufactured under the name "Mexoryl SO" by Chimex,
Terephthalylidenedicamphor sulphonic acid manufactured under the name "Mexoryl SX" by Chimex,
Polyacrylamidomethylbenzylidenecamphor manufactured under the name "Mexoryl SW" by Chimex.

### Phenylbenzimidazole derivatives:

Phenylbenzimidazole sulphonic acid sold in particular under the trade name "Eusolex 232" by Merck,
Disodium phenyl dibenzimidazole tetra sulphonate sold under the trade name "Neo Heliopan AP" by Haarmann and Reimer.

### Phenylbenzotriazole derivatives:

Drometrizole trisiloxane sold under the name "Silatrizole" by Rhodia Chimie,
Methylenebis(benzotriazolyl)tetramethylbutylphenol sold in solid form under the trade name "MIXXIM BB/100" by Fairmount Chemical, or in micronized form as an aqueous dispersion under the trade name "Tinosorb M" by Ciba Specialty Chemicals.

### Triazine derivatives:

- bis-Ethylhexyloxyphenol Methoxyphenyl Triazine sold under the trade name "Tinosorb S" by Ciba Geigy,
- Ethylhexyltriazone sold in particular under the trade name "Uvinul T150" by BASF,
- Diethylhexylbutamidotriazone sold under the trade name "Uvasorb HEB" by Sigma 3V,
- 2,4,6-tris(dineopentyl 4'-aminobenzalmalonate)-s-triazine,
- 2,4,6-tris(diisobutyl 4'-aminobenzalmalonate)-s-triazine,
- 2,4-bis(n-butyl 4'-aminobenzoate)-6-aminopropyltrisiloxane-s-triazine,
- 2,4-bis(dineopentyl 4'-aminobenzalmalonate)-6-(n-butyl 4'-aminobenzoate)-s-triazine,
- symmetrical triazine screening agents described in patent US 6,225,467, patent application WO 2004/085412 (see compounds 6 and 9) or the document "Symmetrical Triazine Derivatives" IP.COM Journal, IP.COM Inc., West Henrietta, NY, US (20 September 2004), especially 2,4,6-tris(biphenyl-1,3,5-triazines (in particular 2,4,6-tris(biphenyl-4-yl)-1,3,5-triazine and 2,4,6-tris(terphenyl)-1,3,5-triazine, which is included in patent applications WO 06/035000, WO 06/034982, WO 06/034991, WO 06/035007, WO 2006/034992 and WO 2006/034985.

### Anthranilic derivatives:

Menthyl anthranilate sold under the trade name "Neo Heliopan MA" by Haarmann and Reimer.

### Imidazoline derivatives:

Ethylhexyl dimethoxybenzylidenedioxoimidazolinepropionate.

### Benzalmalonate derivatives:

Polyorganosiloxane containing benzalmalonate functions, for instance Polysilicone-15, sold under the trade name "Parsol SLX" by Hoffmann LaRoche.

### 4,4-Diarylbutadiene derivatives:

1,1-Dicarboxy(2,2'-dimethylpropyl)-4,4-diphenyl-butadiene.

### Benzoxazole derivatives:

2,4-bis[5-(1-dimethylpropyl)benzoxazol-2-yl(4-phenyl)imino]-6-(2-ethylhexyl)imino-1,3,5-triazine sold under the name Uvasorb K2A by Sigma 3V

Merocyanine derivatives
Octyl-5-N,N-diethylamino-2-phenysulphonyl-2,4-pentadienoate,
and mixtures thereof.

The preferential organic screening agents are chosen from:
Ethylhexyl methoxycinnamate,
Ethylhexyl salicylate,
Homosalate,
Butylmethoxydibenzoylmethane,
Octocrylene,
Phenylbenzimidazole sulphonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphor sulphonic acid,
Disodium phenyldibenzimidazoletetra sulphonate,
Methylenebis-benzotriazolyl tetramethylbutylphenol,
Bis-ethylhexyloxyphenol methoxyphenyl triazine,
Ethylhexyl triazone,
Diethylhexyl butamido triazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(n-butyl 4'-aminobenzoate)-6-aminopropyltrisiloxane-s-triazine,
2,4-Bis(dineopentyl 4'-aminobenzylmalonate)-6-(n-butyl 4'-aminobenzoate)-s-triazine,
2,4,6-Tris(biphenyl-4-yl)-1,3,5-triazine, 2,4,6-Tris(terphenyl)-1,3,5-triazine,
Drometrizole trisiloxane,
Polysilicone-15,
1,1-Dicarboxy(2,2'-dimethylpropyl)-4,4-diphenyl-butadiene,
2,4-Bis[5-(1-dimethylpropyl)benzoxazol-2-yl(4-phenyl)imino]-6-(2-ethylhexyl)imino-1,3,5-triazine, Octyl-5-N,N-diethylamino-2-phenylsulphonyl-2,4-pentadienoate
and mixtures thereof.

The inorganic screening agents used in accordance with the present invention are metal oxide pigments. Preferentially the inorganic UV screening agents of the invention are metal oxide particles having an average elementary particle size of less than or equal to 500 nm, more preferentially between 5 nm and 500 nm, and more preferentially still between 10 nm and 100 nm.

They may be selected especially from titanium oxides, zinc oxides, iron oxides, zirconium oxides, cerium oxides or mixtures thereof.

Coated or uncoated metal oxide pigments of this kind are described more particularly in the patent application EP-A-0 518 773. Commercial pigments that may be mentioned include the products sold by the companies Kemira, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or of aluminium), polyethylene, silicones, proteins (collagen, elastin), alkanolamines, silicon oxides, metal oxides or sodium hexametaphosphate.

The coated pigments are more particularly titanium oxides that have been coated:
- with silica, such as the product "Sunveil" from the company Ikeda,
- with silica and iron oxide, such as the product "Sunveil F" from the company Ikeda,
- with silica and alumina, such as the products "Microtitanium Dioxide MT 500 SA" and "Microtitanium Dioxide MT 100 SA" from the company Tayca and "Tioveil" from the company Tioxide,
- with alumina, such as the products "Tipaque TTO-55 (B)" and "Tipaque TTO-55 (A)" from the company Ishihara and "UVT 14/4" from the company Kemira,
- with alumina and aluminium stearate, such as the products "Microtitanium Dioxide MT 100 T, MT 100 TX, MT 100 Z and MT-01" from the company Tayca, the products "Solaveil CT-10 W", "Solaveil CT 100" and "Solaveil CT200", from the company Croda and the product "Eusolex T-AVO" from the company Merck,
- with silica, alumina and alginic acid, such as the product "MT-100 AQ" from the company Tayca,
- with alumina and aluminium laurate, such as the product "Microtitanium Dioxide MT 100 S" from the company Tayca,
- with iron oxide and iron stearate, such as the product "Microtitanium Dioxide MT 100 F" from the company Tayca,
- with zinc oxide and zinc stearate, such as the product "BR 351" from the company Tayca,
- with silica and alumina and treated with a silicone, such as the products "Microtitanium Dioxide MT 600 SAS", "Microtitanium Dioxide MT 500 SAS" or "Microtitanium Dioxide MT 100 SAS" from the company Tayca,
- with silica, alumina and aluminium stearate and treated with a silicone, such as the product "STT-30-DS" from the company Titan Kogyo,
- with silica and treated with a silicone, such as the product "UV-Titan X 195" from the company Kemira,
- with alumina and treated with a silicone, such as the products "Tipaque TTO-55 (S)" from the company Ishihara or "UV Titan M 262" from the company Kemira,
- with triethanolamine, such as the product "STT-65-S" from the company Titan Kogyo,
- with stearic acid, such as the product "Tipaque TTO-55 (C)" from the company Ishihara,
- with sodium hexametaphosphate, such as the product "Microtitanium Dioxide MT 150 W" from the company Tayca.
- TiO₂ treated with octyltrimethylsilane sold under the trade name "T 805" by the company Degussa Silices,
- TiO₂ treated with a polydimethylsiloxane sold under the trade name "70250 Cardre UF TiO2SI3" by the company Cardre,
- anatase/rutile TiO₂ treated with a polydimethylhydrogenosiloxane sold under the trade name "Microtitanium Dioxide USP Grade Hydrophobic" by the company Color Techniques.

The uncoated titanium oxide pigments are sold, for example, by the company Tayca under the trade names "Microtitanium Dioxide MT 500 B" or "Microtitanium Dioxide MT 600 B", by the company Degussa under the name "P 25", by the company Wackher under the name "Transparent titanium oxide PW", by the company Miyoshi Kasei under the name "UFTR", by the company Tomen under the name "ITS" and by the company Tioxide under the name "Tioveil AQ".

The uncoated zinc oxide pigments are, for example:
- those sold under the name "Z-Cote" by the company Sunsmart;
- those sold under the name "Nanox" by the company Elementis;
- those sold under the name "Nanogard WCD 2025" by the company Nanophase Technologies.

The coated zinc oxide pigments are, for example:
- those sold under the name "Zinc Oxide CS-5" by the company Toshibi (ZnO coated with polymethylhydrogensiloxane);
- those sold under the name "Nanogard Zinc Oxide FN" by the company Nanophase Technologies (as a 40% dispersion in Finsolv TN, C₁₂-C₁₅ alkyl benzoate);
- those sold under the name "Daitopersion Zn-30" and "Daitopersion Zn-50" by the company Daito (dispersions in cyclopolymethylsiloxane/oxyethylenated polydimethylsiloxane, containing 30% or 50% of nanozinc oxides coated with silica and polymethylhydrogensiloxane);

- those sold under the name "NFD Ultrafine ZnO" by the company Daikin (ZnO coated with perfluoroalkyl phosphate and copolymer based on perfluoroalkylethyl as a dispersion in cyclopentasiloxane);
- those sold under the name "SPD-Z1" by the company Shin-Etsu (ZnO coated with silicone-grafted acrylic polymer, dispersed in cyclodimethylsiloxane);
- those sold under the name "Escalol Z100" by the company ISP (alumina-treated ZnO dispersed in an ethylhexyl methoxycinnamate/PVP-hexadecene/methicone copolymer mixture);
- those sold under the name "Fuji ZnO-SMS-10" by the company Fuji Pigment (ZnO coated with silica and polymethylsilsesquioxane);
- those sold under the name "Nanox Gel TN" by the company Elementis (ZnO dispersed at a concentration of 55% in C₁₂-C₁₅ alkyl benzoate with hydroxystearic acid polycondensate).

The uncoated cerium oxide pigments may be for example those sold under the name "Colloidal Cerium Oxide" by the company Rhone-Poulenc.
The uncoated iron oxide pigments are sold, for example, by the company Arnaud under the names "Nanogard WCD 2002 (FE 45B)", "Nanogard Iron FE 45 BL AQ", "Nanogard FE 45R AQ" and "Nanogard WCD 2006 (FE 45R)" or by the company Mitsubishi under the name "TY-220",

The coated iron oxide pigments are sold, for example, by the company Arnaud under the names "Nanogard WCD 2008 (FE 45B FN)", "Nanogard WCD 2009 (FE 45B 556)", "Nanogard FE 45 BL 345" and "Nanogard FE 45 BL" or by the company BASF under the name "Transparent Iron Oxide".

Mention may also be made of mixtures of metal oxides, especially of titanium dioxide and of cerium dioxide, including the silica-coated equal-weight mixture of titanium dioxide and of cerium dioxide, sold by the company Ikeda under the name "Sunveil A", and also the alumina-, silica- and silicone-coated mixture of titanium dioxide and of zinc dioxide, such as the product "M 261" sold by the company Kemira, or the alumina-, silica- and glycerol-coated mixture of titanium dioxide and of zinc dioxide, such as the product "M 211" sold by the company Kemira.

According to the invention, coated or uncoated titanium oxide pigments are particularly preferred.

The photoprotective system according to the invention is preferably present in the compositions according to the invention in a content ranging from 0.1% to 40% by weight and in particular from 5% to 25% by weight relative to the total weight of the composition.

The compositions in accordance with the present invention may also comprise standard cosmetic adjuvants chosen especially from fatty substances, organic solvents, ionic or nonionic, hydrophilic or lipophilic thickeners, demulcents, humectants, opacifiers, stabilizers, emollients, silicones, antifoams, fragrances, preserving agents, anionic, cationic, nonionic, zwitterionic or amphoteric surfactants, polymers, acidifying or basifying agents or any other ingredient usually used in cosmetics and/or dermatology.

The fatty substances may consist of an oil or a wax other than the apolar waxes as defined above, or mixtures thereof. The term "oil" means a compound that is liquid at room temperature. The term "wax" means a compound that is solid or substantially solid at room temperature and whose melting point is generally greater than 35°C.

Oils that may be mentioned include mineral oils (paraffin); plant oils (sweet almond oil, macadamia oil, grapeseed oil or jojoba oil); synthetic oils, for instance perhydrosqualene, fatty alcohols or fatty amides (for instance isopropyl lauroyl sarcosinate sold under the name "Eldew SL-205" by the company Ajinomoto), fatty acids or fatty esters (for instance the C₁₂-C₁₅ alkyl benzoate sold under the trade name "Finsolv TN" or "Witconol TN" by the company Witco, octyl palmitate, isopropyl lanolate and triglycerides, including capric/caprylic acid triglycerides, and dicaprylyl carbonate sold under the name "Cetiol CC" by the company Cognis), oxyethylenated or oxypropylenated fatty esters and ethers; silicone oils (cyclomethicone and polydimethylsiloxanes, or PDMS) or fluoro oils, and polyalkylenes.

Waxy compounds that may be mentioned include carnauba wax, beeswax, hydrogenated castor oil, polyethylene waxes and polymethylene waxes, for instance the product sold under the name Cirebelle 303 by the company Sasol.

Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.

Hydrophilic thickeners that may be mentioned include carboxyvinyl polymers such as the Carbopol products (carbomers) and the Pemulen products (acrylate/C₁₀-C₃₀-alkylacrylate copolymer); polyacrylamides, for instance the crosslinked copolymers sold under the names Sepigel 305 (CTFA name: polyacrylamide/C13-14 isoparaffin/ Laureth 7) or Simulgel 600 (CTFA name: acrylamide/sodium acryloyldimethyltaurate copolymer/isohexadecane/polysorbate 80) by the company SEPPIC; 2-acrylamido-2-methylpropane sulphonic acid polymers and copolymers, which are optionally crosslinked and/or neutralized, for instance the poly(2-acrylamido-2-methylpropane sulphonic acid) sold by the company Hoechst under the trade name "Hostacerin AMPS" (CTFA name: ammonium polyacryloyldimethyltaurate) or Simulgel 800 sold by the company SEPPIC (CTFA name: sodium polyacryloyldimethyltaurate/polysorbate 80/sorbitan oleate); copolymers of 2-acrylamido-2-methylpropane sulphonic acid and of hydroxyethyl acrylate, for instance Simulgel NS and Sepinov EMT 10 sold by the company SEPPIC; cellulose-based derivatives such as hydroxyethylcellulose; polysaccharides and especially gums such as xanthan gum; and mixtures thereof.

Lipophilic thickeners that may be mentioned include synthetic polymers such as poly(C₁₀-C₃₀ alkyl acrylates) sold under the name "Intelimer IPA 13-1" and "Intelimer IPA 13-6" by the company Landec, or modified clays such as hectorite and its derivatives, for instance the products sold under the Bentone names.

As will be appreciated, a person skilled in the art will take care to select the optional additional compound(s) mentioned above and/or the amounts thereof such that the advantageous properties intrinsically associated with the compositions in accordance with the invention are not, or are not substantially, adversely affected by the envisaged addition(s).

The compositions according to the invention contain an aqueous phase and preferably a continuous aqueous phase and are especially in the form of a lotion or serum; in the form of aqueous suspensions; in the form of an aqueous gel, in the form of a simple or complex (W/O/W) oil/water or water/oil emulsion such as a milk, a cream or a cream-gel.

They may optionally be packaged as an aerosol and may be in the form of a mousse or spray. They may also be in a solid form such as sticks.

The compositions according to the invention are preferably in the form of an oil-in-water emulsion.

When it is an emulsion, the aqueous phase of this emulsion may comprise a nonionic vesicular dispersion prepared according to known processes (Bangham, Standish and Watkins, J. Mol. Biol. 13, 238 (1965), FR 2 315 991 and FR 2 416 008).

When it is an emulsion, the composition may be stablized:
- by gelling agents and/or thickeners for instance the hydrodispersions as described in patents EP 683 661 and EP 787 483;
- or else by inorganic or organic fillers having a size of less than 200 nm with an amphiphilic character such as the emulsions of Pickering type as described in patent applications EP 987 001, EP 987 002, EP 987 003, EP 987 004, EP 987 005, EP 986 006;
- and by surfactants or emulsifiers.

For the O/W emulsions, examples of emulsifiers that may be mentioned include nonionic emulsifiers such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters, for instance the mixture PEG-100 stearate/glyceryl stearate sold, for example, by the company ICI under the name Arlacel 165; oxyalkylenated (oxyethylenated and/or oxypropylenated) fatty alkyl ethers; sugar esters, for instance sucrose stearate; fatty alkyl ethers of sugars, especially polyalkylglucosides (APG) such as decylglucoside and laurylglucoside sold, for example, by the company Henkel under the respective names Plantaren 2000 and Plantaren 1200, cetostearyl glucoside optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of a mixture of arachidyl alcohol, behenyl alcohol and arachidyl glucoside, sold under the name Montanov 202 by the company SEPPIC. According to one particular embodiment of the invention, the mixture of the alkylpolyglucoside as defined above with the corresponding fatty alcohol may be in the form of a self-emulsifying composition as described, for example, in document WO-A-92/06778.

Among the other emulsion stabilizers that will be used more particularly are isophthalic acid or sulphoisophthalic acid polymers, and in particular phthalate/ sulphoisophthalate/glycol copolymers, for example the diethylene glycol/phthalate/isophthalate/1,4-cyclohexanedimethanol copolymer (INCI name: Polyester-5) sold under the name "Eastman AQ Polymer" (AQ35S, AQ38S, AQ55S and AQ48 Ultra) by the company Eastman Chemical.

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists in the use of the compositions according to the invention as defined above for the manufacture of cosmetic products for treating the skin, the lips, the nails, the hair, the eyelashes, the eyebrows and/or the scalp, especially care products, sun protection products and makeup products.

The cosmetic compositions according to the invention may be used, for example, as makeup products.

The compositions according to the invention may additionally, further, comprise additional cosmetic and dermatological active agents.

It will be possible especially to choose the additional active agents from moisturizers, desquamating agents, agents for improving the barrier function, depigmenting agents, antioxidants, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing the activity of the sebaceous glands, agents for stimulating the energy metabolism of cells, tensioning agents, lipid restructuring agents, slimming agents, agents for promoting the cutaneous microcirculation, calmatives and/or anti-irritants, sebo-regulating or antiseborrhoeic agents, astringents, cicatrizing agents, anti-inflammatory agents, anti-acne agents and agents which promote natural colouring of the skin.

A person skilled in the art will select the said active agent or agents as a function of the desired effect on the skin, hair, eyelashes, eyebrows or nails.

For caring for and/or making up skin which has aged, he or she will preferably select at least one active agent chosen from moisturizers, desquamating agents, agents for improving the barrier function, depigmenting agents, antioxidants, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing the activity of the sebaceous glands, agents for stimulating the energy metabolism of cells, lipid restructuring agents, agents promoting the cutaneous microcirculation for the area around the eyes and agents which promote the natural colouring of the skin.

For caring for and/or making up greasy skin, the person skilled in the art will preferably select at least one active agent chosen from desquamating agents, sebo-regulating or antiseborrhoeic agents and astringents.

### 1. Moisturizers or humectants

Moisturizers or humectants that may especially be mentioned include glycerol and derivatives thereof, urea and derivatives thereof, especially Hydrovance^{®} sold by National Starch, lactic acid, hyaluronic acid, AHAs, BHAs, sodium pidolate, xylitol, serine, sodium lactate, ectoin and derivatives thereof, chitosan and derivatives thereof, collagen, plankton, an extract of *Imperata cylindra* sold under the name Moist 24^{®} by the company Sederma, acrylic acid homopolymers, for instance Lipidure-HM^{®} from NOF Corporation, beta-glucan and in particular sodium carboxymethyl beta-glucan from Mibelle-AG-Biochemistry; a mixture of passionflower oil, apricot oil, corn oil and rice bran oil sold by Nestle under the name NutraLipids^{®}; a C-glycoside derivative such as those described in patent application WO 02/051 828 and in particular C-β-D-xylopyranoside-2-hydroxypropane in the form of a solution containing 30% by weight of active material in a water/propylene glycol mixture (60/40% by weight) such as the product sold by Chimex under the trade name Mexoryl SBB^{®}; an oil of musk rose sold by Nestle; an extract of the microalga *Prophyridium cruentum* enriched with zinc, sold by Vincience under the name Algualane Zinc^{®}; spheres of collagen and of chondroitin sulphate of marine origin (Atelocollagen) sold by the company Engelhard Lyon under the name Marine Filling Spheres; hyaluronic acid spheres such as those sold by the company Engelhard Lyon; and arginine.

The moisturizer that will preferably be used is chosen from urea and derivatives thereof, especially Hydrovance^{®} sold by National Starch, hyaluronic acid, AHAs, BHAs, acrylic acid homopolymers, for instance Lipidure-HM^{®} from NOF Corporation, beta-glucan and in particular sodium carboxymethyl beta-glucan from Mibelle-AG-Biochemistry; a mixture of passionflower oil, apricot oil, corn oil and rice bran oil sold by Nestle under the name NutraLipids^{®}; a C-glycoside derivative such as those described in patent application WO 02/051 828 and in particular C-β-D-xylopyranoside-2-hydroxypropane in the form of a solution containing 30% by weight of active material in a water/propylene glycol mixture (60/40% by weight) such as the product sold by Chimex under the trade name Mexoryl SBB^{®}; an oil of musk rose sold by Nestle; an extract of the microalga *Prophyridium cruentum* enriched with zinc, sold by Vincience under the name Algualane Zinc^{®}; spheres of collagen and of chondroitin sulphate of marine origin (Atelocollagen) sold by the company Engelhard Lyon under the name Marine Filling Spheres; hyaluronic acid spheres such as those sold by the company Engelhard Lyon; and arginine.

### 2. Desquamating agents

The term "desquamating agent" means any compound capable of acting:
- either directly on desquamation by promoting exfoliation, such as β-hydroxy acids (BHA), in particular salicylic acid and derivatives thereof (including 5-n-octanoylsalicylic acid, also known as capryloyl salicylic acid as the INCI name); α-hydroxy acids (AHA), such as glycolic acid, citric acid, lactic acid, tartaric acid, malic acid or mandelic acid; 8-hexadecene-1,16-dicarboxylic acid or 9-octadecenedioic acid; urea and derivatives thereof; gentisic acid and derivatives thereof; oligofucoses; cinnamic acid; *Saphora japonica* extract; resveratrol, and certain jasmonic acid derivatives;

- or on the enzymes involved in the desquamation or degradation of corneodesmosomes, glycosidases, stratum corneum chymotryptic enzyme (SCCE) or other proteases (trypsin, chymotrypsin-like). Mention may be made of amino sulphonic compounds and in particular 4-(2-hydroxyethyl)piperazine-1-propane sulphonic acid (HEPES); 2-oxothiazolidine-4-carboxylic acid (procysteine) and derivatives thereof; derivatives of α-amino acids of glycine type (as described in EP-0 852 949, and also sodium methyl glycine diacetate sold by BASF under the trade name Trilon M); honey; sugar derivatives such as O-octanoyl-6-D-maltose and N-acetylglucosamine.

As other desquamating agents that may be used in the composition according to the invention, mention may be made of:
- oligofructoses, EDTA and derivatives thereof, laminaria extracts, o-linoleyl-6D-glucose, (3-hydroxy-2-pentylcyclopentyl)acetic acid, glycerol trilactate, O-octanyl-6'-D-maltose S-carboxymethylcysteine, siliceous derivatives of salicylate such as those described in patent EP 0 796 861, oligofucases such as those described in patent EP 0 218 200, 5-acyl salicylic acid salts, active agents with effects on transglutaminase, as in patent EP 0 899 330,
- extract of the flowers of ficus *Opuntia indica* such as Exfolactive® from Silab,
- 8-hexadecene-1,16-dicarboxylic acid,
- esters of glucose and of vitamin F, and
- mixtures thereof.

Preferred desquamating agents that may be mentioned include β**-**hydroxy acids such as 5-n-octanoyl salicylic acid; urea; glycolic acid, citric acid, lactic acid, tartaric acid, malic acid or mandelic acid; 4-(2-hydroxyethyl)piperazine-1-propane sulphonic acid (HEPES); extract of *Saphora japonica;* honey; N-acetyl glucosamine; sodium methyl glycine diacetate, and mixtures thereof.

Even more preferentially, a desquamating agent chosen from 5-n-octanoyl salicylic acid; urea; 4-(2-hydroxyethyl)piperazine-1-propane sulphonic acid (HEPES); extract of *Saphora japonica;* honey; N-acetyl glucosamine; sodium methyl glycine diacetate, and mixtures thereof, will be used in the compositions of the invention.

### 3. Agents for improving the barrier function

As agents for improving the barrier function, mention may be made especially of arginine, serine, an extract of *Thermus thermophilus* such as Venuceane^{®} from Sederma, an extract of the rhizome of wild yarn (*Dioscorea villosa*) such as Actigen Y^{®} from Active Organics, plankton extracts, for instance Omega Plankton^{®} from Secma, yeast extracts, for instance Relipidiur^{®} from Coletica, a chestnut extract such as Recoverine^{®} from Silab, a cedar bud extract such as Gatuline Zen^{®} from Gattefossé, sphingosines, for instance salicyloyl sphingosine sold under the name Phytosphingosine^{®} SLC by the company Degussa, a mixture of xylitol, polyxylityl glycoside and xylitan, for instance Aquaxyl^{®} from SEPPIC, extracts of Solanacea plants, for instance Lipidessence^{®} from Coletica, omega 3 unsaturated oils such as oils of musk rose; and mixtures thereof. Mention may also be made especially of ceramides or derivatives thereof, in particular ceramides of type 2 (for instance N-oleoyldihydrosphingosine), of type 3 (for instance stearoyl-4-hydroxysphinganine, as the INCI name) and of type 5 (for instance N-2-hydroxypalmitoyldihydrosphingosine, having the INCI name: hydroxypalmitoyl sphinganine), sphingoid-based compounds, glycosphingolipids, phospholipids, cholesterol and derivatives thereof, phytosterols, essential fatty acids, diacylglycerol, 4-chromanone and chromone derivatives, petroleum jelly, lanolin, shea butter, cocoa butter, lanolin and PCA salts.

As preferred agents having a restructuring effect on the cutaneous barrier, mention will be made of an extract of *Thermus thermophilus,* an extract of wild yarn rhizome (*Dioscorea villosa*)*,* a yeast extract, a chestnut extract, a cedar bud extract, arginine, serine, ceramides especially of type 3 and 5; and mixtures thereof.

Serine, arginine or a mixture thereof will preferably be used.

### 4. Depigmenting agents

Depigmenting agents that may especially be mentioned include vitamin C and derivatives thereof and especially vitamin CG, CP and 3-0 ethyl vitamin C, alpha and beta arbutin, ferulic acid, lucinol and derivatives thereof, kojic acid, resorcinol and derivatives thereof, tranexamic acid and derivatives thereof, gentisic acid, homogentisate, methyl gentisate or homogentisate, dioic acid, calcium D-pantheteine sulphonate, lipoic acid, ellagic acid, vitamin B3, linoleic acid and derivatives thereof, ceramides and homologues thereof, plant derivatives, for instance camomile, bearberry, the aloe family (vera, ferox, bardensis), mulberry or skullcap; a kiwi fruit (*Actinidia chinensis*) juice sold by Gattefossé, an extract of *Paeonia suffruticosa* root, such as the product sold by the company Ichimaru Pharcos under the name Botanpi Liquid B^{®} , an extract of brown sugar (*Saccharum officinarum*)*,* such as the molasses extract sold by the company Taiyo Kagaku under the name Molasses Liquid, without this list being exhaustive.

As preferred depigmenting agents, use will be made of vitamin C and its derivatives and especially vitamin CG, vitamin CP and 3-0-ethyl-vitamin C, alpha- and beta-arbutin, ferulic acid, kojic acid, resorcinol and its derivatives, calcium D-pantetheine sulphonate, lipoic acid, ellagic acid, vitamin B3, a kiwi fruit juice (*Actinidia chinensis*) sold by Gattefossé, an extract of *Paeonia suffruticosa* root such as that sold by Ichimaru Pharcos under the name BOTANPI LIQUID B^{®}.

### 5. Antioxidants

Mention may be made especially of tocopherol and esters thereof, in particular tocopherol acetate; ascorbic acid and derivatives thereof, in particular magnesium ascorbyl phosphate and ascorbyl glucoside; ferulic acid; serine; ellagic acid, phloretin, polyphenols, tannins, tannic acid, epigallocatechins and natural extracts containing them, anthocyans, rosemary extracts, olive leaf extracts, for instance those from the company Silab, green tea extracts, resveratrol and derivatives thereof, ergothioneine, N-acetylcysteine, an extract of the brown alga *Pelvetia canaliculata,* for instance Pelvetiane^{®} from Secma, chlorogenic acid, biotin, chelating agents, such as BHT and BHA, N,N'-bis(3,4,5-trimethoxybenzyl)ethylenediamine and salts thereof; idebenone, plant extracts, for instance Pronalen Bioprotect TM from the company Provital; coenzyme Q10, bioflavonoids, SODs, phytantriol, lignans, melatonin, pidolates, glutathione, caprylyl glycol, phloretin, Totarol™ or extract of *Podocarpus totara* containing Totarol (totara-8,11,13-trienol or 2-phenanthrenol, 4b,5,6,7,8,8a,9,10-octahydro-4b,8,8-trimethyl-1-(1-methylethyl)-; a jasmine extract such as the product sold by Silab under the name Helisun^{®}; hesperitin laurate such as Flavagrum PEG^{®} from the company Engelhard Lyon; an extract of *Paeonia suffruticosa* root, such as the product sold by the company Ichimaru Pharcos under the name Botanpi Liquid B^{®} a lychee extract such as the lychee pericarp extract sold by the company Cognis under the name Litchiderm LS 9704^{®}, a pomegranate extract (*Punica granatum*)*,* such as the product sold by the company Draco Natural Products.

Other anti-ageing agents that may be mentioned include DHEA and derivatives thereof, boswellic acid, rosemary extracts, carotenoids (**β**-carotene, zeaxanthin and lutein), cysteic acid, copper derivatives and jasmonic acid.

Preferred antioxidants that will especially be used include ferulic acid; serine; phloretin, a pomegranate extract, biotin, chelating agents such as BHT, BHA, N,N'-bis(3,4,5-trimethoxybenzyl)ethylenediamine and salts thereof; caprylyl glycol, phloretin, Totarol™, a jasmine extract such as the product sold by Silab under the name Helisun^{®}; hesperitin laurate such as Flavagrum PEG^{®} from the company Engelhard Lyon; an extract of *Paeonia suffruticosa* root, such as the product sold by the company Ichimaru Pharcos under the name Botanpi Liquid B^{®}.

### 6. Dermo-relaxing or dermo-decontracting agents

Examples that may be mentioned include manganese gluconate and other salts, adenosine, alverine citrate and salts thereof, glycine, an extract of *Iris pallida,* a hexapeptide (Argeriline R from Lipotec) or sapogenins, for instance wild yarn and the carbonyl amines described in patent application EP 1 484 052. Examples of sapogenins that may be mentioned include those described in patent application WO 02/47650, in particular wild yarn, the diosgenin extracted especially from *Dioscorea opposita* or any extract naturally containing or containing after treatment one or more sapogenins (wild yarn rhizome, agave leaf, which contains hecogenin and tigogenin, extracts of Liliacea plants and more particularly yucca or smilax containing smilagenin and sarsapogenin, or sarsaparilla root) or Actigen Y from the company Actives Organics; or ginger.

Mention may also be made of DMAE (dimethyl MEA), extracts of sea fennel, of rockrose, of helichrysum, of anise, of paracress, and an extract of *Acmella oleracea,* for instance Gatuline^{®} from Gattefossé.

Preferred dermo-relaxing agents that will be mentioned include adenosine, manganese gluconate, wild yarn, sea fennel, glycine and alverine.

### 7. Anti-glycation agents

The term "anti-glycation agent" means a compound that prevents and/or reduces the glycation of skin proteins, in particular dermal proteins such as collagen.

Anti-glycation agents that may especially be mentioned include extracts of plants of the Ericacea family, such as an extract of blueberry (*vaccinium angustifolium* or *vaccinium myrtillus*)*,* for example the product sold under the name Blueberry Herbasol Extract PG by the company Cosmetochem, ergothioneine and derivatives thereof, hydroxystilbenes and derivatives thereof, such as resveratrol and 3,3',5,5'-tetrahydroxystilbene (these anti-glycation agents are described in patent applications FR 2 802 425, FR 2 810 548, FR 2 796 278 and FR 2 802 420, respectively), dihydroxystilbenes and derivatives thereof, polypeptides of arginine and of lysine such as the product sold under the name Amadorine^{®} by the company Solabia, carcinine hydrochloride (sold by Exsymol under the name Alistin^{®}), an extract of *Helianthus annuus,* for instance Antiglyskin^{®} from Silab, wine extracts such as the extract of powdered white wine on a maltodextrin support sold under the name Vin blanc déshydraté 2F by the company Givaudan, thioctic acid (or alpha-lipoic acid), a mixture of extract of bearberry and of marine glycogen, for instance Aglycal LS 8777^{®} from Laboratoires Serobiologiques, and an extract of black tea, for instance Kombuchka^{®} from Sederma, and mixtures thereof.

Preferred anti-glycation agents that will be mentioned include extracts of blueberry (*Vaccinium myrtillus*) and extract of black tea.

### 8. Agents for stimulating the synthesis of dermal and/or epidermal macromolecules and/or for preventing their degradation

Among the active agents for stimulating the dermal macromolecules or for preventing their degradation, mention may be made of those acting:
- either on collagen synthesis, such as extracts of *Centella asiatica,* asiaticosides and derivatives thereof; ascorbic acid or vitamin C and derivatives thereof; synthetic peptides such as iamin, biopeptide CL or palmitoyl oligopeptide sold by the company Sederma; peptides extracted from plants, such as the soybean hydrolysate sold by the company Coletica under the trade name Phytokine^{®}; rice peptides such as Nutripeptide^{®} from Silab, methylsilanol mannuronate such as Algisium C^{®} sold by Exsymol; plant hormones such as auxins and lignans; folic acid; and an extract of *Medicago sativa* (alfalfa) such as the product sold by Silab under the name Vitanol^{®}; a peptide extract of hazelnut such as the product sold by the company Solabia under the name Nuteline C^{®}; and arginine;
- or on the inhibition of collagen degradation, in particular agents acting on the inhibition of metalloproteases (MMP) more particularly such as MMP 1, 2, 3 and 9. Mention may be made of: retinoids and derivatives, extracts of *Medicago sativa* such as Vitanol^{®} from Silab, an extract of *Aphanizomenon flosaquae* (Cyanophyceae) sold under the name Lanablue^{®} by Atrium Biotechnologies, oligopeptides and lipopeptides, lipoamino acids, the malt extract sold by the company Coletica under the trade name Collalift^{®}; blueberry or rosemary extracts; lycopene; isoflavones, derivatives thereof or plant extracts containing them, in particular extracts of soybean (sold, for example, by the company Ichimaru Pharcos under the trade name Flavosterone SB^{®}), of red clover, of flax or of kakkon; an extract of lychee such as the lychee pericarp extract sold by the company Cognis under the name Litchiderm LS 9704^{®}; Dipalmitoyl Hydroxyproline sold by SEPPIC under the name Sepilift DPHP^{®}: *Baccharis genistelloide* or Baccharine sold by Silab, an extract of moringa such as Arganyl LS 9781^{®} from Cognis; the sage extract described in patent application FR-A-2 812 544 from the Labiatae family (*Salvia officinalis* from the company Flacksmann), an extract of rhododendron, a blueberry extract, and an extract of *Vaccinium myrtillus* such as those described in patent application FR-A-2 814 950;
- or on the synthesis of molecules belonging to the elastin family (elastin and fibrillin), such as: retinol and derivatives, in particular retinyl palmitate; the extract of *Saccharomyces cerevisiae* sold by the company LSN under the trade name Cytovitin^{®}; and the extract of the alga *Macrocystis pyrifera* sold by the company Secma under the trade name Kelpadelie^{®}; a peptide extract of hazelnut such as the product sold by the company Solabia under the trade name Nuteline C^{®};
- or on inhibition of elastin degradation, such as the peptide extract of seeds of *Pisum sativum* sold by the company LSN under the trade name Parelastyl^{®}; heparinoids; and the N-acylamino amide compounds described in patent application WO 01/94381, such as {2-[acetyl(3-trifluoromethylphenyl)amino]-3-methyl-butyrylamino}acetic acid, also known as N-[N-acetyl, N'-(3-trifluoromethyl)phenylvalyl]glycine, or N-acetyl-N-[3-(trifluoromethyl) phenyl] valyl glycine or acetyl trifluoromethyl phenyl valylglycine, or an ester thereof with a C₁-C₆ alcohol; an extract of rice peptides such as Colhibin^{®} from Pentapharm, or an extract of *Phyllanthus emblica* such as Emblica^{®} from Rona;
- or on the synthesis of glycosaminoglycans, such as the product of fermentation of milk with *Lactobacillus vulgaris,* sold by the company Brooks under the trade name Biomin Yoghurt^{®}; the extract of the brown alga *Padina pavonica* sold by the company Alban Muller under the trade name HSP3^{®}; the *Saccharomyces cerevisiae* extract available especially from the company Silab under the trade name Firmalift^{®} or from the company LSN under the trade name Cytovitin^{®}; an extract of *Laminaria ochroleuca* such as Laminaine^{®} from Secma; essence of Mamaku from Lucas Meyer, and an extract of cress (Odraline^{®} from Silab);
- or on the synthesis of fibronectin, such as the extract of the zooplankton Salina sold by the company Seporga under the trade name GP4G^{®}; the yeast extract available especially from the company Alban Muller under the trade name Drieline^{®}; and the palmitoyl pentapeptide sold by the company Sederma under the trade name Matrixil^{®}.

Among the active agents for stimulating epidermal macromolecules, such as fillagrin and keratins, mention may be made especially of the extract of lupin sold by the company Silab under the trade name Structurine^{®}; the extract of *Fagus sylvatica* beech buds sold by the company Gattefossé under the trade name Gatuline^{®} RC; and the extract of the zooplankton Salina sold by the company Seporga under the trade name GP4G^{®}; the copper tripeptide from Procyte; a peptide extract of *Voandzeia substerranea* such as the product sold by the company Laboratoires Serobiologiques under the trade name Filladyn LS 9397^{®}.

Preferably, an active agent that stimulates the synthesis of dermal and/or epidermal macromolecules and/or that prevents their degradation, chosen from agents for stimulating the synthesis of glycosaminoglycans, agents for inhibiting elastin degradation, agents for stimulating fibronectin synthesis, agents for stimulating the synthesis of epidermal macromolecules, and mixtures thereof, will be used.

Even more preferentially, an active agent that stimulates the synthesis of the glycosaminoglycans, chosen from an extract of the brown alga *Padina pavonica,* an extract of *Saccharomyces cerevisiae,* an extract of *Laminaria ochroleuca,* essence of Mamaku, and an extract of cress, and mixtures thereof, will even more preferentially be used.

As preferred active agents for stimulating the synthesis of dermal and/or epidermal macromolecules and/or for preventing their degradation, mention may be made of:
synthetic peptides such as iamin, the biopeptide CL or palmitoyloligopeptide sold by the company Sederma; peptides extracted from plants, such as the soybean hydrolysate sold by the company Coletica under the trade name Phytokine^{®}; rice peptides such as Nutripeptide^{®} from Silab, methylsilanol mannuronate such as Algisium C^{®} sold by Exsymol; folic acid; an extract of *Medicago sativa* (alfalfa), such as the product sold by Silab under the name Vitanol^{®}; a peptide extract of hazelnut, such as the product sold by the company Solabia under the name Nuteline C^{®}; arginine; an extract of *Aphanizomenon flos-aquae* (Cyanophyceae) sold under the name Lanablue^{®} by Atrium Biotechnologies, the malt extract sold by the company Coletica under the trade name Collalift^{®}, lycopene; an extract of lychee; an extract of moringa such as Arganyl LS 9781^{®} from Cognis; an extract of *Vaccinium myrtillus* such as those described in patent application FR-A-2 814 950; retinol and derivatives thereof, in particular retinyl palmitate; the extract of *Saccharomyces cerevisiae* sold by the company LSN under the trade name Cytovitin^{®}; a peptide extract of hazelnut such as the product sold by the company Solabia under the name Nuteline C^{®}; {2-[acetyl(3-trifluoromethylphenyl)amino]-3-methylbutyrylamino}-acetic acid, also known as N-[N-acetyl, N'-(3-trifluoromethyl)phenylvalyl]glycine, or N-acetyl-N-[3-(trifluoromethyl)phenyl]valylglycine or acetyl trifluoromethyl phenyl valylglycine, or an ester thereof with a C₁-C₆ alcohol; an extract of rice peptides such as Colhibin^{®} from Pentapharm, or an extract of *Phyllanthus emblica* such as Emblica^{®} from Rona; the extract of the brown alga *Padina pavonica* sold by the company Alban Muller under the trade name HSP3^{®}; the extract of *Saccharomyces cerevisiae* available especially from the company Silab under the trade name Firmalift^{®} or from the company LSN under the trade name Cytovitin^{®}; an extract of *Laminaria ochroleuca* such as Laminaine^{®} from Secma; the essence of Mamaku from Lucas Meyer, the extract of lupin sold by the company Silab under the trade name Structurine^{®}; the extract of *Fagus sylvatica* beech buds sold by the company Gattefossé under the trade name Gatuline^{®} RC.

### 9. Agents for stimulating fibroblast or keratinocyte proliferation and/or keratinocyte differentiation

The agents for stimulating fibroblast proliferation that may be used in the composition according to the invention may be chosen, for example, from plant proteins or polypeptides, extracted especially from soybean (for example a soybean extract sold by the company LSN under the name Eleseryl SH-VEG 8^{®} or sold by the company Silab under the trade name Raffermine^{®}); an extract of hydrolysed soybean proteins such as Ridulisse^{®} from Silab; and plant hormones such as gibberellins and cytokinins; a peptide extract of hazelnut such as the product sold by the company Solabia under the name Nuteline C^{®}.

Preferably, an agent that promotes keratinocyte proliferation and/or differentiation will be used.

The agents for stimulating keratinocyte proliferation that may be used in the composition according to the invention especially comprise adenosine; phloroglucinol, the extract of *Hydrangea macrophylla* leaves, for instance Amacha Liquid E^{®} from Ichimaru Pharcos, a yeast extract such as Stimoderm^{®} from CLR; the extract of *Larrea divaricata* such as Capislow^{®} from Sederma, mixtures of extracts of papaya, of olive leaves and of lemon, such as Xyleine^{®} from Vincience, the extract of *Hydrangea macrophylla* leaves, for instance Amacha Liquid E^{®} from Ichimaru Pharcos, retinol and esters thereof, including retinyl palmitate, phloroglucinol, the nut cake extracts sold by the Gattefossé and the extracts of *Solanum tuberosum* such as Dermolectine^{®} sold by Sederma.

Among the agents for stimulating keratinocyte differentiation are, for example, minerals such as calcium; sea fennel, a peptide extract of lupin, such as the product sold by the company Silab under the trade name Structurine^{®}; sodium beta-sitosteryl sulphate, such as the product sold by the company Seporga under the trade name Phytocohesine^{®}; and a water-soluble extract of corn, such as the product sold by the company Solabia under the trade name Phytovityl^{®}; a peptide extract of *Voandzeia substerranea* such as the product sold by the company Laboratoires Serobiologiques under the trade name Filladyn LS 9397^{®}; and lignans such as secoisolariciresinol, and retinol and esters thereof, including retinyl palmitate.

As agents for stimulating keratinocyte proliferation and/or differentiation, mention may also be made of oestrogens such as oestradiol and homologues; cytokines.

As preferred active agents for stimulating fibroblast or keratinocyte proliferation and/or keratinocyte differentiation, mention will be made of plant proteins or polypeptides, extracted especially from soybean (for example a soybean extract sold by the company LSN under the name Eleseryl SH-VEG 8^{®} or sold by the company Silab under the trade name Raffermine^{®}); an extract of hydrolysed soybean proteins such as Ridulisse^{®} from Silab; a peptide extract of hazelnut such as the product sold by the company Solabia under the name Nuteline C^{®}; adenosine; phloroglucinol, a yeast extract such as Stimoderm^{®} from CLR; a peptide extract of lupin such as the product sold by the company Silab under the trade name Structurine^{®}; a water-soluble corn extract, such as the product sold by the company Solabia under the trade name Phytovityl^{®}; a peptide extract of *Voandzeia substerranea,* such as the product sold by the company Laboratoires Serobiologiques under the trade name Filladyn LS 9397^{®}; retinol and esters thereof, including retinyl palmitate.

### 10. Agents for promoting the maturation of the horny envelope

Agents that participate in the maturation of the horny envelope, which becomes impaired with age and induces a decrease in transglutaminase activity, may be used in the compositions of the invention. Examples that may be mentioned include urea and derivatives thereof and in particular Hydrovance^{®} from National Starch and the other active agents mentioned in L'Oréal patent application FR 2 877 220 (unpublished).

### 11. NO-synthase inhibitors

The agent with an inhibitory action on NO synthase may be chosen from OPCs (procyannidol oligomers); plant extracts of the species *Vitis vinifera* sold especially by the company Euromed under the name "Leucocyanidines de raisins extra", or by the company Indena under the name Leucoselect^{®}, or finally by the company Hansen under the name "Extrait de marc de raisin"; plant extracts of the species *Olea europaea* preferably obtained from olive tree leaves and sold especially by the company Vinyals in the form of a dry extract, or by the company Biologia & Technologia under the trade name Eurol^{®} BT; and plant extracts of the species *Gingko biloba,* preferably a dry aqueous extract of this plant sold by the company Beaufour under the trade name "Ginkgo biloba extrait standard", and mixtures thereof.

### 12. Peripheral benzodiazepine receptor (PBR) antagonists

Mention may be made, for example, of 1-(2-chlorophenyl)-N-(1-methylpropyl)-3-isoquinoline carboxamide; the compounds described in patent applications WO 03/030 937 and WO 03/068 753, pyridazino[4,5-b]indole-1-acetamide derivatives of general formula (VII) as described in document WO 00/44384.

### 13. Agents for increasing the activity of the sebaceous glands

Mention may be made, for example, of methyl dehydrojasmonate, hecogenin, hedione and O-linoleyl-6D-glucose, and mixtures thereof.

### 14. Agents for stimulating the energy metabolism of cells

The active agent for stimulating the energy metabolism of cells may be chosen, for example, from biotin, an extract of *Saccharomyces cerevisiae* such as Phosphovital^{®} from Sederma, the mixture of sodium, manganese, zinc and magnesium salts of pyrrolidonecarboxylic acid, for instance Physiogenyl^{®} from Solabia, a mixture of zinc, copper and magnesium gluconate, such as Sepitonic M3^{®} from SEPPIC, and mixtures thereof; a beta-glucan derived from *Saccharomyces cerevisiae,* such as the product sold by the company Mibelle AG Biochemistry.

### 15. Lipid restructuring agents

By `lipid restructuring agents' are meant, according to the invention, agents capable of stimulating lipogenesis and of promoting adipocyte differentiation, thereby making it possible to prevent or retard the wasting of fat contained in the skin-supporting tissues, otherwise known as 'wasting of the fat structure of the skin'.
By 'fat structure of the skin' is meant the network of fat cells which form the volumes on which the skin of the face rests and to which it moulds.

These agents are intended for reducing the loss of skin density and/or the wasting of the fat structure of the skin, more particularly on the cheeks and in the area around the eye, and/or preventing the collapse and/or hollowing of the volumes of the face, the loss of consistency of the skin and/or its maintenance, more particularly on the cheeks and around the eye, and/or improving the underlying volumes of the skin of the face and/or neck, more particularly on the cheeks, the oval of the face, and the area around the eye, and/or improving the density, the springiness and the maintenance of the skin, more particularly on the cheeks, the oval of the face and the area around the eye, and/or reshaping the facial features, in particular the oval of the face.

Examples of lipid restructuring agents include especially a black tea extract, such as the extract of fermented black tea that is sold by Sederma under the name Kombuchka®, and an *Artemisia abrotanum* extract, such as that sold by Silab under the name Pulpactyl®.

### 16. Slimming agents

Slimming (lipolytic) agents that may especially be mentioned include caffeine, theophylline and its derivatives, theobromine, sericosine, asiatic acid, acefylline, aminophylline, chloroethyltheophylline, diprofylline, diniprophylline, etamiphylline and its derivatives, etofylline and proxyphylline; extracts of tea, of coffee, of guarana, of mate, of cola (*Cola nitida*) and especially the dry extract of guarana fruit (*Paulina sorbilis*) containing 8% to 10% caffeine; extracts of climbing ivy (*Hedera helix*), of arnica (*Arnica montana L*), of rosemary (*Rosmarinus officinalis N*), of marigold (*Calendula officinalis*), of sage (*Salvia officinalis L*), of ginseng (*Panax ginseng*), of St. John's wort (*Hypericum perforatum*), of butcher's-broom (*Ruscus aculeatus L*)*,* of meadowsweet (*Filipendula ulmaria L*)*,* of orthosiphon (*Orthosiphon stamincus Benth*), of birch (*Betula alba*)*,* of pumpwood and of argan tree, extracts of ginkgo biloba, extracts of horsetail, extracts of escin, extracts of cangzhu, extracts of *Chrysanthellum indicum,* extracts of diosgenin-rich Dioscorea plants or pure diosgenin or hecogenin and derivatives thereof, extracts of Ballota, extracts of *Guioa,* of *Davallia,* of *Terminalia,* of *Barringtonia,* of *Trema* or of *Antirobia,* the extract of bitter orange pips; an extract of husks of cocoa beans (*Theobroma cacao*) such as the product sold by Solabia under the name Caobromine^{®}.

### 17. Agents for promoting the cutaneous microcirculation

The active agent acting on the cutaneous microcirculation may be used for preventing dulling of the complexion and/or for improving the appearance of the area around the eye, especially for reducing shadows. It may be chosen, for example, from an extract of maritime pine bark, for instance Pycnogenol^{®} from Biolandes, manganese gluconate (Givobio GMn^{®} from SEPPIC), an extract of *Ammi visnaga* such as Visnadine from Indena, extract of lupin (Eclaline^{®} from Silab), the protein coupling of hydrolysed wheat/palmitic acid with palmitic acid, such as Epaline 100 from Laboratoires Carilène, the extract of bitter orange blossom (Remoduline^{®} from Silab), vitamin P and derivatives thereof, for instance methyl-4 esculetol sodium monoethanoate sold under the name Permethol^{®} by the company Sephytal, extracts of Ruscus, of common horse chestnut, of ivy, of ginseng and of melilot, caffeine, nicotinate and derivatives thereof, lysine and derivatives thereof, for instance Asparlyne^{®} from Solabia, an extract of black tea such as Kombuchka from Sederma; rutin salts; an extract of the alga *Corallina officinalis,* such as the product sold by Codif; and mixtures thereof.

As preferred agents for promoting the cutaneous microcirculation, mention will be made of caffeine, an extract of bitter orange blossom, an extract of black tea, rutin salts and an extract of the alga *Corallina officinalis.*

### 18. Calmatives or anti-irritants

The term "calmative" means a compound that can reduce the sensation of stinging, itching or tautness of the skin.

As calmatives that may be used in the composition according to the invention, mention may be made of: procyannidol oligomers, vitamins E, C, B5 and B3, caffeine and derivatives thereof, pentacylic triterpenes and plant extracts containing them, β-glycyrrhetinic acid and salts or derivatives thereof (stearyl glycyrrhetate, 3-stearoyloxyglycyrrhetic acid or glycyrrhetinic acid monoglucuronide) and also plants containing them (e.g.: *Glycyrrhiza glabra*), oleanolic acid and salts thereof, ursolic acid and salts thereof, boswellic acid and salts thereof, betulinic acid and salts thereof, an extract of *Paeonia suffruticosa* and/or *lactiflora,* an extract of *Laminaria saccharina,* extracts of *Centella asiatica,* Canola oil, bisabolol, the phosphoric diester of vitamin E and C, for instance Sepivital EPC^{®} from SEPPIC, camomile extracts, allantoin, omega-3 unsaturated oils such as musk rose oil, blackcurrant oil, Ecchium oil, fish oil or beauty-leaf oil, plankton extracts, capryloyl glycine, a mixture of water lily blossom extract and of palmitoylproline, such as the product sold under the name Seppicalm VG^{®} by the company SEPPIC, an extract of *Boswellia serrata,* an extract of *Centipeda cunninghami,* such as the product sold under the name Cehami PF^{®} by the company TRI-K Industries, an extract of sunflower seeds, in particular Helioxine^{®} from Silab, an extract of *Linum usitatissimum* seeds, for instance Sensiline^{®} from Silab, tocotrienols, piperonal, an extract of *Epilobium angustifolium,* such as the product sold under the name Canadian Willowherb Extract by the company Fytokem Products, *Aloe vera,* phytosterols, cornflower water, rose water, an extract of mint, in particular of mint leaves, for instance Calmiskin^{®} from Silab, anise derivatives, filamentous bacteria, for instance *Vitreoscilla filiformis* as described in patent EP 761 204 and sold by Chimex under the name Mexoryl SBG^{®}, an extract of rose petals, for instance Rose Flower Herbasol^{®} extract from the company Cosmetochem, shea butter, a mixture of the waxy fraction of barley seeds obtained by supercritical CO₂, of shea butter and of argan oil, for instance Stimu-tex AS^{®} from Pentapharm, alkaline-earth metal salts, especially of strontium, a fermented extract of Alteromonas sold under the name Abyssine® by the company Atrium Biotechnologies; spring water from the Vichy basin, such as waters originating from the Celestins, Chomel, Grande-Grille, Hôpital, Lucas and Parc sources, and preferably water from the Lucas source; an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

As preferred calmatives according to the invention, use will be made of:
β-glycyrrhetinic acid and salts or derivatives thereof (stearyl glycyrrhetate, 3-stearoyloxyglycyrrhetic acid or glycyrrhetinic acid monoglucuronide) and also plants containing them (e.g. *Glycyrrhiza glabra*); ursolic acid and salts thereof; extracts of *Centella asiatica,* Canola oil, bisabolol; camomile extracts, allantoin; a mixture of extract of water lily blossom and of palmitoylproline, such as the product sold under the name Seppicalm VG^{®} by the company SEPPIC; *Aloe vera,* rose water, extract of mint, in particular of mint leaves, such as Calmiskin^{®} from Silab, filamentous bacteria such as *Vitreoscilla filiformis* as described in patent EP 761 204 and sold by Chimex under the name Mexoryl SBG^{®}, an extract of rose petals such as Rose Flower Herbasol^{®} extract from the company Cosmetochem, shea butter, a fermented extract of Alteromonas sold under the name Abyssine® by the company Atrium Biotechnologies; spring water from the Vichy basin, such as waters originating from the Celestins, Chomel, Grande-Grille, Hôpital, Lucas and Parc sources, and preferably water from the Lucas source; an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

### 19. Sebo-regulating or anti-seborrhoeic agents

The term "sebo-regulating or anti-seborrhoeic agents" especially means agents capable of regulating the activity of the sebaceous glands.

Mention may be made especially of:
- retinoic acid, benzoyl peroxide, sulphur, vitamin B6 (or pyridoxine), selenium chloride and sea fennel;
- mixtures of extract of cinnamon, of tea and of octanoylglycine such as Sepicontrol A5 TEA^{®} from SEPPIC;
- the mixture of cinnamon, sarcosine and octanoylglycine sold especially by the company SEPPIC under the trade name Sepicontrol A5^{®};
- zinc salts such as zinc gluconate, zinc pyrrolidonecarboxylate (or zinc pidolate), zinc lactate, zinc aspartate, zinc carboxylate, zinc salicylate and zinc cysteate;
- copper derivatives and in particular copper pidolate such as Cuivridone^{®} from Solabia;
- extracts of plants of the species *Arnica montana, Cinchona succirubra, Eugenia caryophyllata, Humulus lupulus, Hypericum perforatum, Mentha piperita, Rosmarinus officinalis, Salvia oficinalis and Thymus vulgaris,* all sold, for example, by the company Maruzen;
- extracts of meadowsweet (*Spiraea ulmaria*)*,* such as the product sold under the name Sebonormine^{®} by the company Silab;
- extracts of the alga *Laminaria saccharina,* such as the product sold under the name Phlorogine^{®} by the company Biotechmarine;
- mixtures of extracts of salad burnet root (*Sanguisorba officinalis*/*Poterium officinale*), of ginger rhizomes (*Zingiber officinalis*) and of cinnamon bark (*Cinnamomum cassia*), such as the product sold under the name Sebustop^{®} by the company Solabia;
- linseed extracts, such as the product sold under the name Linumine^{®} by the company Lucas Meyer;
- Phellodendron extracts, such as those sold under the name Phellodendron extract BG by the company Maruzen or Oubaku liquid B by the company Ichimaru Pharcos;
- mixtures of argan oil, of *Serenoa serrulata* (saw palmetto) extract and of sesame seed extract, such as the product sold under the name Regu SEB^{®} by the company Pentapharm;
- mixtures of extracts of willowherb, of *Terminalia chebula,* of nasturtium and of bioavailable zinc (microalgae), such as the product sold under the name Seborilys^{®} by the company Green Tech;
- extracts of *Pygeum afrianum,* such as the product sold under the name Pygeum afrianum sterolic lipid extract by the company Euromed;
- extracts of *Serenoa serrulata,* such as the products sold under the name Viapure Sabal by the company Actives International or those sold by the company Euromed;
- mixtures of extracts of plantain, of *Berberis aquifolium* and of sodium salicylate, such as the product sold under the name Seboclear^{®} by the company Rahn;
- clove extract, such as the product sold under the name Clove extract powder by the company Maruzen;
- argan oil, such as the product sold under the name Lipofructyl^{®} by Laboratoires Serobiologiques;
- lactic protein filtrates, such as the product sold under the name Normaseb^{®} by the company Sederma;
- extracts of the alga *Laminaria,* such as the product sold under the name Laminarghane^{®} by the company Biotechmarine;
- oligosaccharides of the alga *Laminaria digitata,* such as the product sold under the name Phycosaccharide AC by the company Codif;
- sugar cane extracts, such as the product sold under the name Policosanol^{®} by the company Sabinsa;
- sulphonated shale oil, such as the product sold under the name Ichthyol Pale^{®} by the company Ichthyol;
- European meadowsweet (*Spiraea ulmaria*) extracts, such as the product sold under the name Cytobiol^{®} Ulmaire by the company Libiol;
- sebacic acid, especially sold in the form of a sodium polyacrylate gel under the name Sebosoft^{®} by the company Sederma;
- glucomannans extracted from konjac tuber and modified with alkyl sulphonate chains, such as the product sold under the name Biopol Beta by the company Arch Chemical;
- extracts of *Sophora angustifolia,* such as those sold under the name Sophora powder or Sophora extract by the company Bioland;
- extracts of *Cinchona succirubra* bark, such as the product sold under the name Red Bark HS by the company Alban Muller;
- extracts of *Quillaja saponaria*, such as the product sold under the name Panama wood HS by the company Alban Muller;
- glycine grafted onto an undecylenic chain, such as the product sold under the name Lipacide UG OR by the company SEPPIC;
- the mixture of oleanolic acid and of nordihydroguaiaretic acid, such as the product sold in the form of a gel under the name AC.Net by the company Sederma;
- phthalimidoperoxyhexanoic acid;
- tri(C₁₂-C₁₃) alkyl citrate sold under the name Cosmacol^{®} ECI by the company Sasol; tri(C₁₄-C₁₅) alkyl citrate sold under the name Cosmacol^{®} ECL by the company Sasol;
- 10-hydroxydecanoic acid, and especially mixtures of 10-hydroxydecanoic acid, of sebacic acid and of 1,10-decanediol, such as the product sold under the name Acnacidol^{®} BG by the company Vincience; and
- mixtures thereof.

Preferred anti-seborrhoeic active agents that may be mentioned include:
- benzoyl peroxide and vitamin B6 (or pyridoxine),
- zinc salts such as zinc gluconate, zinc pyrrolidonecarboxylate (or zinc pidolate), zinc lactate, zinc aspartate, zinc carboxylate, zinc salicylate and zinc cysteate;
- meadowsweet (*Spiraea ulmaria*) extracts, such as the product sold under the name Sebonormine^{®} by the company Silab;
- extracts of the alga *Laminaria saccharina,* such as the product sold under the name Phlorogine^{®} by the company Biotechmarine;
- mixtures of extracts of salad burnet root (*Sanguisorba officinalis*/*Poterium officinale),* of ginger rhizomes (*Zingiber officinalis*) and of cinnamon bark (*Cinnamomum cassia*), such as the product sold under the name Sebustop^{®} by the company Solabia;
- clove extract, such as the product sold under the name Clove extract powder by the company Maruzen;
- lactic protein filtrates, such as the product sold under the name Normaseb^{®} by the company Sederma;
- European meadowsweet (*Spiraea ulmaria*) extracts, such as the product sold under the name Cytobiol^{®} Ulmaire by the company Libiol;
- sebacic acid, especially sold in the form of a sodium polyacrylate gel under the name Sebosoft^{®} by the company Sederma;
- glycine grafted onto an undecylenic chain, such as the product sold under the name Lipacide UG OR by the company SEPPIC;
- tri (C₁₂-C₁₃) alkyl citrate sold under the name Cosmacol^{®} ECI by the company Sasol; tri (C₁₄-C₁₅) alkyl citrate sold under the name Cosmacol^{®} ECL by the company Sasol;
- 10-hydroxydecanoic acid, and especially mixtures of 10-hydroxydecanoic acid, of sebacic acid and of 1,10-decanediol, such as the product sold under the name Acnacidol^{®} BG by the company Vincience; and
- mixtures thereof.

Preferentially, the anti-seborrhoeic active agent is chosen from:
- zinc salts such as zinc gluconate, zinc pyrrolidonecarboxylate (or zinc pidolate), zinc lactate, zinc aspartate, zinc carboxylate, zinc salicylate and zinc cysteate; and preferably zinc pyrrolidonecarboxylate (or zinc pidolate) or zinc salicylate;
- clove extract, such as the product sold under the name Clove extract powder by the company Maruzen;
- glycine grafted onto an undecylenic chain, such as the product sold under the name Lipacide UG OR by the company SEPPIC;
- tri(C₁₂**-**C₁₃)alkyl citrate sold under the name Cosmacol^{®} ECI by the company Sasol; tri(C₁₄**-**C₁₅)alkyl citrate sold under the name Cosmacol^{®} ECL by the company Sasol;
- and mixtures thereof.

The anti-seborrhoeic active agent is, for example, present in a content ranging from 0.1% to 10% by weight, preferably from 0.1% to 5% by weight and preferentially from 0.5% to 3% by weight relative to the total weight of the composition.

### 20. Astringents

According to the invention, the term "astringents" means agents for combating the dilation of the sebaceous follicles.

As astringents that may be used in the composition according to the invention, mention may be made of extracts of mushroom pulp (*Polyporus officinalis),* for instance Laricyl LS8865^{®} from Cognis, extracts of *Terminalia catappa* and *Sambucus nigra,* for instance Phytofirm LS9120^{®} from Cognis, extracts of gall nut, for instance Tanlex VE^{®} from Ichimaru Pharcos, aluminium hydroxychloride, centella extracts (e.g. Plantactiv centella from Cognis), dicetyl dimethylammonium chloride, for instance Varisoft 432 CG^{®} from Degussa, common horsechestnut extracts, mallow extracts, witch-hazel extracts, sweet almond extracts, marsh mallow root extracts and linseed extracts, for instance Almondermin LS 3380^{®} from Cognis, burdock extracts, nettle extracts, birch extracts, horsetail extracts, camomile extracts, for instance those sold under the name Extrapone 9 special^{®} by the company Symrise, skullcap extracts, European meadowsweet extracts (for example Cytobiol Ulmaire from Libiol), a mixture of extracts of white ginger, of horsetail, of nettle, of rosemary and of yucca, for instance Herb extract B1348^{®} from Bell Flavors & Fragrances, extracts of acacia, of elm, of white willow, of cinnamon, of birch and of meadowsweet, Panama sapogenins, zinc phenol sulphonate from Interchemical, extracts of gentian, of cucumber and of walnut, the mixture of extracts of Ratanhia, of grapefruit, of gumweed and of oak gall, for instance Epilami^{®} from Alban Muller.

As preferred astringents according to the invention, use will be made of skullcap extracts, European meadowsweet extracts, meadowsweet extracts, gentian extracts and burdock extracts, and mixtures thereof.

### 21. Cicatrizing agents

Examples of cicatrizing agents that may especially be mentioned include:
allantoin, urea, certain amino acids, for instance hydroxyproline, arginine, and serine, and also extracts of white lily (for instance Phytélène Lys 37EG 16295 from Indena), a yeast extract, for instance the cicatrizing agent LS LO/7225B from Laboratoires Sérobiologiques), tamanu oil, extract of *Saccharomyces cerevisiae,* for instance Biodynes® TRF® from Arch Chemical, oat extracts, chitosan and derivatives, for instance chitosan glutamate, carrot extracts, artemia extract, for instance GP4G® from Vincience, sodium acexamate, lavandin extracts, propolis extracts, ximeninic acid and salts thereof, rose hip oil, marigold extracts, for instance Souci Ami® Liposolible from Alban Muller, horsetail extracts, lemon peel extracts, for instance Herbasol® citron from Cosmetochem, helichrysum extracts, common yarrow extracts and folic acid.

As preferred cicatrizing agents according to the invention, use will be made of arginine, serine, folic acid, tamanu oil, sodium acexamate, horsetail extracts and helichrysum extracts, and mixtures thereof.

### 22. Anti-inflammatory agents

As particular anti-inflammatory agents that may be used according to the invention, mention may be made of cortisone, hydrocortisone, indomethacin, betamethasone, azelaic acid, acetaminophen, diclofenac, clobetasol propionate, folic acid; an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

Preferred anti-inflammatory agents that will be mentioned are azelaic acid, folic acid, an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

### 23. Anti-acne agents

In one advantageous aspect of the invention, the composition may also comprise at least one active anti-acne agent.

The term "active anti-acne agent" especially means any active agent that has effects on the specific flora of greasy skin, for instance *Propionibacterium acnes (P. acnes) .*

These effects may be bactericidal.

Antibacterial agents that may especially be mentioned include:
- actives and preserving agents with antimicrobial activity as mentioned in patent application DE 103 24 567, which is incorporated into the present invention by reference,
- asiatic acid,
- the monoethanolamine salt of 1-hydroxy-4-methyl 6-trimethylpentyl-2-pyridone (INCI name: piroctone olamine), sold especially under the name Octopirox^{®} by the company Clariant;
- citronellic acid, perillic acid (or 4-isopropenylcyclohex-1-enecarboxylic acid),
- glyceryl 2-ethylhexyl ether (INCI name: ethylhexylglycerine), for example sold under the name Sensiva SC 50^{®} by the company Shulke & Mayr,
- glyceryl caprylate/caprate, for example sold under the name Capmul MCM^{®} by the company Abitec;
- sodium calcium phosphosilicate, especially sold under the names Bioactive Glasspowder^{®} and Actysse Premier BG^{®} by the company Schott Glass;
- silver-based particles, for example those sold under the name Metashine ME 2025 PS^{®} by the company Nippon Sheet Glass;
- hop cone extract (*Humulus lupulus*) obtained by supercritical CO₂ extraction, such as the product sold under the name HOP CO2-TO extract^{®} by the company Flavex Naturextrakte,
- St.-John's Wort extract obtained by supercritical CO₂ extraction, such as the product sold under the name St.-John's Wort CO2-TO extract^{®} by the company Flavex Naturextrakte,
- the mixture of extracts of roots of *Scutellaria baicalensis,* of *Paeonia suffruticosa* and *Glycyrrhiza glabra,* such as the product sold under the name BMB - CF^{®} by the company Naturogin,
- argan tree extract, for instance Argapure LS9710^{®} from Cognis;
- bearberry leaf extracts, for instance the product sold under the name Melfade-J by the company Pentapharm;
- 10-hydroxy-2-decanoic acid such as Acnacidol P^{®} from Vincience, sodium ursolate, azelaic acid, diiodomethyl p-tolyl sulphone such as Amical Flowable^{®} from Angus, malachite powder, zinc oxide such as Zincare^{®} from Elementis GMBH, octadecenedioic acid such as Arlatone dioic DCA^{®} from Uniqema; ellagic acid; 2,4,4'-trichloro-2'-hydroxydiphenyl ether (or triclosan), 1-(3',4'-dichlorophenyl)-3-(4'-chlorophenyl)urea (or triclocarban), 3,4,4'-trichlorocarbanilide, 3',4',5'-trichlorosalicylanilide, phenoxyethanol, phenoxypropanol, phenoxyisopropanol, hexamidine isethionate, metronidazole and salts thereof, miconazole and salts thereof, itraconazole, terconazole, econazole, ketoconazole, saperconazole, fluconazole, clotrimazole, butoconazole, oxiconazole, sulphaconazole, sulconazole, terbinafine, ciclopirox, ciclopiroxolamine, undecylenic acid and salts thereof, benzoyl peroxide, 3-hydroxybenzoic acid, 4-hydroxybenzoic acid, phytic acid, N-acetyl-L-cysteine, lipoic acid, azelaic acid and salts thereof, arachidonic acid, resorcinol, 3,4,4'-trichloro-carbanalide, octoxyglycerine or octoglycerine, octanoylglycine such as Lipacid C8G^{®} from SEPPIC, caprylyl glycol, 10-hydroxy-2-decanoic acid, dichlorophenylimidazoldioxolane and derivatives thereof described in patent application WO 93/18743, iodopropynyl butylcarbamate, 3,7,11-trimethyldodeca-2,5,10-trienol or farnesol, phytosphingosines; quaternary ammonium salts, for instance cetyltrimethylammonium salts and cetylpyridinium salts, and
- mixtures thereof.

Mention may also be made of certain surfactants with an antimicrobial effect, for instance sodium cocoamphoacetate or disodium diacetate such as Miranol C2M Conc. NP, betaines, for instance the cocoyl betaine Genagen KB from Clariant, sodium lauryl ether sulphate, for instance Emal 270 D from Kao, decyl glucoside, for instance Plantacare 2000 UP, branched C₁₂₋₁₃ dialkyl malates, for instance Cosmacol EMI, propylene glycol monoesters, for instance propylene glycol monolaurate, monocaprylate or monocaprate, lauryldimethylamine betaine, for instance Empigen BB/LS, and also polyquaternary ammoniums such as Quaternium-24 or Bardac 2050 from Lonza and those described in patent FR 0 108 283, and mixtures thereof.

As preferred antimicrobial agents, an agent chosen from octoglycerine or octoxyglycerine, and 10-hydroxy-2-decanoic acid, and mixtures thereof, will be used in the compositions of the invention.

Other additional active anti-acne agents may be added to the abovementioned active anti-acne agents.

Mention may be made especially of active agents with bacterial anti-adhesion effects or agents that act on the biofilm of bacteria to prevent them from multiplying.

As agents for preventing and/or reducing the adhesion of microorganisms, mention may be made especially of: phytantriol and derivatives thereof as described in patent application EP 1 529 523, plant oils such as wheatgerm oil, calendula oil, castor oil, olive oil, avocado oil, sweet almond oil, groundnut oil, jojoba oil, sesame seed oil, apricot kernel oil, sunflower oil and macadamia oil, described in patent EP 1 133 979, or certain surfactants such as disodium cocoamphodiacetate, oxyethylenated (7 EO) glyceryl cocoate, 18-hexadecenyl succinate, octoxyglyceryl palmitate, octoxyglyceryl behenate, dioctyl adipate, PPG-15 stearyl ether, and the branched C₁₂-C₁₃ dialkyl tartrate described in patent EP 1 129 694, and mixtures thereof.

In particular with regard to the propagation of *P. acnes,* or as active agents that act on the biofilm of bacteria to prevent them from proliferating, mention may be made of pentylene glycol, Nylon-66 (polyamide 66 fibres), rice bran oil, polyvinyl alcohol such as Celvol 540 PV alcohol^{®} from Celanese Chemical, rapeseed oil such as Akorex L^{®} from Karlshamns, and fructose derivatives, and mixtures thereof.

The anti-acne active agent may be present in a content ranging from 0.01% to 10% by weight and preferably from 0.05% to 5% by weight relative to the total weight of the composition.

As a function of the nature and/or solubility of the abovementioned active agents, a person skilled in the art will know how to select the most suitable embodiment according to the invention.

As lipophilic active agents that may be used in at least one of the compositions of the invention, mention may be made especially of D-α-tocopherol, DL-α-tocopherol, D-α-tocopheryl acetate, DL-α-tocopheryl acetate, ascorbyl palmitate, vitamin F glycerides, D vitamins, vitamin D2, vitamin D3, retinol, retinol esters, retinyl palmitate, retinyl propionate, carotenes including β-carotene, D-panthenol, farnesol, farnesyl acetate, salicylic acid and derivatives thereof, for instance 5-n-octanoylsalicylic acid, α-hydroxy acid alkyl esters such as citric acid, lactic acid, glycolic acid, asiatic acid, madecassic acid, asiaticoside, the total extract of *Centella asiatica,* β-glycyrrhetinic acid, α-bisabolol, ceramides, for instance 2-oleoylamino-1,3-octadecane, phytantriol, phospholipids of marine origin rich in polyunsaturated essential fatty acids, ethoxyquine, rosemary extract, balm extract, quercetin, extract of dried microalgae, essential oil of bergamot, octyl methoxycinnamate, butylmethoxydibenzoylmethane, octyl triazone, 3,5-di-tert-butyl-4-hydroxy-3-benzylidenecamphor, antibiotics, antifungal agents, anaesthetics, analgesics, antiseptics, antiviral agents, pesticides and herbicides, and mixtures thereof.

### 24. Agents for promoting the naturally pinkish coloration of the skin

Mention may be made especially of:
- a self-tanning agent, i.e. an agent which, when applied to the skin, especially to the face, can produce a tan effect that is more or less similar in appearance to that which may result from prolonged exposure to the sun (natural tan) or under a UV lamp;
- an additional colouring agent, i.e. any compound that has a particular affinity for the skin, which allows it to give the skin a lasting, non-covering coloration (i.e. that does not have a tendency to opacify the skin) and that is not removed either with water or using a solvent, and that withstands both rubbing and washing with a solution containing surfactants. Such a lasting coloration is thus distinguished from the superficial and transient coloration provided, for example, by a makeup pigment; and mixtures thereof.

The self-tanning agents are generally chosen from monocarbonyl or polycarbonyl compounds, for instance isatin, alloxan, ninhydrin, glyceraldehyde, mesotartaric aldehyde, glutaraldehyde, erythrulose, pyrazoline-4,5-dione derivatives as described in patent application FR 2 466 492 and WO 97/35842, dihydroxyacetone (DHA) and 4,4-dihydroxypyrazolin-5-one derivatives as described in patent application EP 903 342. DHA will preferably be used.

The DHA may be used in free and/or encapsulated form, for example in lipid vesicles such as liposomes, especially described in patent application WO 97/25970.

In general, the self-tanning agent is present in an amount ranging from 0.01% to 20% by weight and preferably in an amount of between 0.1% and 10% of the total weight of the composition.

Other dyes that allow modification of the colour produced by the self-tanning agent may also be used.

These dyes may be chosen from synthetic or natural direct dyes.

These dyes may be chosen, for example, from red or orange dyes of the fluoran type such as those described in patent application FR 2 840 806. Mention may be made, for example, of the following dyes:
- tetrabromofluorescein or eosin known under the CTFA name: CI 45380 or Red 21;
- phloxin B known under the CTFA name: CI 45410 or Red 27;
- diiodofluorescein known under the CTFA name: CI 45425 or Orange 10;
- dibromofluorescein known under the CTFA name: CI 45370 or Orange 5;
- the sodium salt of tetrabromofluorescein known under the CTFA name: CI 45380 (Na salt) or Red 22;
- the sodium salt of phloxin B known under the CTFA name: CI 45410 (Na salt) or Red 28;
- the sodium salt of diiodofluorescein known under the CTFA name: CI 45425 (Na salt) or Orange 11;
- erythrosine known under the CTFA name: CI 45430 or Acid Red 51;
- phloxin known under the CTFA name: CI 45405 or Acid Red 98.

These dyes may also be chosen from anthraquinones, caramel, carmine, carbon black, azulene blues, methoxalene, trioxalene, guajazulene, chamuzulene, Bengal rose, cosin 10B, cyanosin and daphinin.

These dyes may also be chosen from indole derivatives, for instance the monohydroxyindoles as described in patent FR 2 651 126 (i.e.: 4-, 5-, 6- or 7-hydroxy-indole) or the dihydroxyindoles as described in patent EP-B-0 425 324 (i.e.: 5,6-dihydroxyindole, 2-methyl-5,6-dihydroxyindole, 3-methyl-5,6-dihydroxyindole or 2,3-dimethyl-5,6-dihydroxyindole).

The cosmetic and/or dermatological active agents will be present in one of the compositions according to the invention in a content ranging from 0.001% to 20% by weight relative to the total weight of the composition, preferably from 0.01% to 10%, more preferably still from 0.5 to 5% and more preferably from 0.1 to 1% by weight relative to the total weight of the composition. The following photoprotective formulations were produced; the amounts are given as weight percentages.

### Examples

Four formulation examples were produced:

Examples No. 1, 2 and 3 are O/W emulsions according to the invention containing lipophilic, hydrophilic and inorganic UV-screening agents, polyamide particles and a starch.

Example No. 4 (outside the invention) is a reference O/W emulsion, the support of which is analogous to that of Example 3, but that does not contain either starch or polyamide.

Examples No. 5, 6, 7 and 8 (outside the invention) are counterexamples, the support of which is analogous to that to that of Example 3 but with fillers other than the gelling starch and the polyamide particles or with starch alone or polyamide particles alone.

The amounts are expressed as weight percentages.

The microscopic and macroscopic appearances are monitored at 24 hours. The stability of the emulsions at 4, 25 and 45°C, after two months, is thus evaluated and a cosmetic evaluation is also carried out, on 3 people, with regard to the following criteria: non-tacky effect, non-greasy effect, bare skin appearance (absence of residual film).

The procedure for preparation of the emulsions is the following:
Phase A₁: The raw materials are mixed and heated at 80°C with stirring for 1/2 h.
Phase A₂: The raw materials are mixed at 25°C.
Phase A: The phases A1 and A2 are added together.
Phase B: The raw materials are mixed and heated at 80°C with stirring.

Phase B is emulsified in Phase A, still with stirring, until a smooth and shiny emulsion is obtained. Finally, Phases C, D and E are added at 25°C.

| **Phase** | **Ingredients** | **Ex 1** | **Ex 2** | **Ex 3** |
|---|---|---|---|---|
| A₁ | Maize starch (Farmal CS 3650 by Corn Products International) | 2.0 | 2.0 | 2.0 |
| | Glycol | 12 | 12 | 12 |
| | Potassium cetyl phosphate (Amphisol K - DSM Nutritional Products) | 1.0 | 1.0 | 1.0 |
| | Xanthan gum (Keltrol CG-T from CP Kelco) | 0.1 | 0.1 | 0.1 |
| | AcrylateS/C₁₀-C₃₀ alkylacrylate crosspolymer (PEMULEN TR1) | 0.25 | 0.25 | 0.25 |
| | Water | qs for 100 | qs for 100 | qs for 100 |
| A₂ | Triethanolamine | 0.71 | 0.71 | 0.71 |
| | Terephthalylidene dicamphor sulfonic acid (MEXORYL SX-CHIMEX) | 0.9 | 0.9 | 0.9 |
| | Water | 5.0 | 5.0 | 5.0 |
| B | Isohexadecane | 4.5 | 4.5 | 9 |
| | Preservative | 1.2 | 1.28 | 1.7 |
| | Synthetic Wax | 1.0 | 1.0 | 1.0 |
| | C12-15 alkyl benzoate | 7.5 | 7.5 | 2.7 |
| | Stearic acid | 1.0 | 1.0 | 1.0 |
| | Mixture of glyceryl mono/distearate and polyethylene glycol stearate (100 EO) (SIMULSOL 165 from SEPPIC) | 1.0 | 1.0 | 1.0 |
| | Butyl methoxy dibenzoylmethane (PARSOL 1789 - DSM Nutritional Products) | 1.5 | 3.0 | 3.0 |
| | Octocrylene (UVINUL N539 - BASF) | 4.0 | 7 | 8.5 |
| | TRI-ANILINO-2,4,6 P-(CARBO ETHYL-2 HEXYL OXY-1') TRI-AZINE-1,3,5 (Uvinul T150 - BASF) | - | 0.5 | - |
| | Aluminium stearate/alumina treated titanium dioxide (MICRO TITANIUM DIOXIDE MT-100 T V) | 1.5 | 3 | 3 |
| | Polydimethylsiloxane (DOW CORNING 200 FLUID 350 CST) | 0.5 | 0.5 | 0.5 |
| C | Nylon-12 powder (ORGASOL 2002 EXD NAT COS from ARKEMA) | 2.5 | 2.5 | 2.5 |
| D | Ethanol | 2.0 | 2.0 | 2.0 |
| E | Fragrance | 0.6 | 0.6 | - |

| **Phase** | **Ingredients** | **Ex 4 (*)** | **Ex 5 (*)** |
|---|---|---|---|
| A₁ | Glycol | 12 | 12 |
| | Potassium cetyl phosphate (Amphisol K - DSM Nutritional Products) | 1.0 | 1.0 |
| | Xanthan gum (Keltrol CG-T from CP Kelco) | 0.1 | 0.1 |
| | AcrylateS/C₁₀-C₃₀ alkylacrylate crosspolymer (PEMULEN TR1) | 0.25 | 0.25 |
| | Water | qs for 100 | qs for 100 |
| A₂ | Triethanolamine | 0.71 | 0.71 |
| | Terephthalylidene dicamphor sulfonic acid (MEXORYL SX-CHIMEX) | 0.9 | 0.9 |
| | Water | 5.0 | 5.0 |
| B | Isohexadecane | 3.95 | 3.95 |
| | Preservative | 1.45 | 1.7 |
| | Polydimethylsiloxane (DOW CORNING 200 FLUID 350 CST) | 0.5 | 0.5 |
| | Synthetic Wax | 1.0 | 1.0 |
| | Vinylpyrrolidone/eicosene copolymer | 1.0 | - |
| | C12-15 alkyl benzoate | 2.7 | 2.7 |
| | Stearic acid | 1.0 | 1.0 |
| | Mixture of glyceryl mono/distearate and polyethylene glycol stearate (100 EO) (SIMULSOL 165 from SEPPIC) | 1.0 | 1.0 |
| | Butyl methoxy dibenzoylmethane (PARSOL 1789 - DSM Nutritional Products) | 3.0 | 3.0 |
| | Octocrylene (UVINUL N539 - BASF) | 8.5 | 8.5 |
| | Aluminium stearate/alumina treated titanium dioxide (MICRO TITANIUM DIOXIDE MT-100 T V) | 3.0 | 3.0 |
| C | Aluminium starch octenylsuccinate (non-gelling starch) | - | 1.5 |
| D | Cyclopentasiloxane (DOW CORNING 245 FLUID) | 5.0 | 5.0 |
| E | Fragrance | 0.6 | - |

| **Phase** | **Ingredients** | **Ex 6 (*)** | **Ex 7 (*)** | **Ex 8 (*)** |
|---|---|---|---|---|
| A₁ | Maize starch (Farmal CS 3650 by Corn Products International) | - | 3.0 | 3.0 |
| | Glycol | 12 | 12 | 12 |
| | Potassium cetyl phosphate (Amphisol K - DSM Nutritional Products) | 1.0 | 1.0 | 1.0 |
| | Xanthan gum (Keltrol CG-T from CP Kelco) | 0.1 | 0.1 | 0.1 |
| | Acrylates/C₁₀-C₃₀ alkylacrylate crosspolymer (PEMULEN TR1) | 0.25 | 0.25 | 0.25 |
| A₂ | Water | qs for 100 | qs for 100 | qs for 100 |
| | Triethanolamine | 0.71 | 0.71 | 0.71 |
| | Terephthalylidene dicamphor sulfonic acid (MEXORYL SX-CHIMEX) | 0.9 | 0.9 | 0.9 |
| | Water | 5.0 | 5.0 | 5.0 |
| B | Isohexadecane | 3.95 | 4.0 | 4.0 |
| | Cyclohexasiloxane (DOW CORNING 245 FLUID) | - | 5 | 5 |
| | Preservative | 1.7 | 1.7 | 1.2 |
| | Polydimethylsiloxane (DOW CORNING 200 FLUID 350 CST) | 0.5 | 0.5 | 0.5 |
| | Synthetic Wax | 1.0 | 1.0 | 1.0 |
| | C12-15 alkyl benzoate | 2.7 | 2.7 | 2.7 |
| | Stearic acid | 1.0 | 1.0 | 1.0 |
| | Mixture of glyceryl mono/distearate and polyethylene glycol stearate (100 EO) (SIMULSOL 165 from SEPPIC) | 1.0 | 1.0 | 1.0 |
| | Butyl methoxy dibenzoylmethane (PARSOL 1789 - DSM Nutritional Products) | 3.0 | 3.0 | 3.0 |
| | Octocrylene (UVINUL N539 - BASF) | 8.5 | 8.5 | 8.5 |
| | Aluminium stearate/alumina treated titanium dioxide (MICRO TITANIUM DIOXIDE MT-100 T V) | 3.0 | 3.0 | 3.0 |
| C | Microbeads of methylsilsesquioxane resin (TOSPEARL 145 A from GE TOSHIBA SILICONES) | 1.0 | - | - |
| | Talc: micronized magnesium silicate (MICRO ACE P3 from NIPPON TALC) | - | - | 2 |
| D | Cyclopentasiloxane (DOW CORNING 245 FLUID) | 5.0 | - | - |
| E | Ethanol | - | 2.0 | 2.0 |

### Tests for evaluation of the stability and of the cosmetic properties

| **Examples** | **Ex 1 (invention)** | **Ex 2 (invention)** | **Ex 3 (invention)** | **Ex 4 (outside of the invention)** | **Ex 5 (outside of the invention)** |
|---|---|---|---|---|---|
| **Stability after 2 months at 4, 25 and 45°C** | Stable | Stable | Stable | Stable | Stable |
| **Cosmetic evaluation** | Non-greasy finish, bare skin effect and non-tacky effect | Non-greasy finish, bare skin effect and non-tacky effect | Non-greasy finish, bare skin effect and non-tacky effect | Greasy, shiny and tacky effect at the end | Greasy, shiny and tacky effect at the end |

| **Examples** | **Ex 6 (outside of the invention)** | **Ex 7 (outside of the invention)** | **Ex 8 (outside of the invention)** |
|---|---|---|---|
| **Stability after 2 months at 4, 25 and 45°C** | Stable | Unstable phase separation | Reagglomeration of the fillers and of the titanium dioxide |
| **Cosmetic evaluation** | More greasy effect | More greasy effect | Greasy, shiny and tacky effect at the end |

### Evaluations of the efficacy and cosmetic performances of Example 2

The performances of Example 2 according to the invention were evaluated and showed SPF and PPD protection values *in vivo* in accordance with the European recommendation for claiming an SPF 30 anti-sun product: the SPF and the PPD protection index were measured according to the methods recommended by COLIPA.

| **Protection index measured** | **Example No. 2** |
|---|---|
| Average SPF (standard deviation) | 33.7 (2.7) |
| PPD (standard deviation) | 18.1 (4.0) |

The sensory evaluation was evaluated on the forearms by a panel of 17 experts.
The method is the following:

The panel of experts trained in describing care products evaluates the formulae for a certain number of descriptors which are graded on a scale of 1 to 15 (low to high).

| **Criterion** | **Formula No. 2** | |
|---|---|---|
| 1 minute after application | | |
| Wet skin | 3.5 | little |
| Fresh film | 7.5 | moderately |

| 2 minutes after application | | |
|---|---|---|
| Shiny skin | 4 | relatively little |
| White skin | 2 | very little |
| Tacky skin | 4 | relatively little |
| Slippery skin | 6.5 | moderately |
| Film-forming | 5 | relatively little |

| 2 minutes after a 2nd application | | |
|---|---|---|
| White skin | 5 | relatively little |

A test under beach usage conditions with Example 2 also made it possible to test the cosmetic appearance and also the comfort and the performance:
A panel of 144 women used the formula.

Results/usage effects and qualities: 90% of women felt that the bare skin was softer and 87% of women found that the formula did not leave their skin tacky and greasy.

## Claims

1. Composition intended for protecting the skin and/or hair against ultraviolet radiation, **characterized by** the fact that it comprises, in a cosmetically acceptable support containing at least one aqueous phase, at least:
(a) a photoprotective system capable of screening out UV radiation;
(b) at least one gelling starch that is not modified by a chemical or physical process which is chosen from unmodified maize starches and
(c) polyamide particles.

2. Composition according to Claim 1, where the gelling starch is such that the amylose/amylopectin weight ratio may vary from 30/70 to 16/84.

3. Composition according to any one of Claims 1 or 2, where the unmodified gelling starch or starches may represent from 0.1 to 10% by weight, preferably from 0.5 to 5% by weight and more particularly from 1 to 3% by weight relative to total weight of the final composition.

4. Composition according to any one of Claims 1 to 3, where the polyamide particles are particles of polyamide PA-12 or polyamide PA-6.

5. Composition according to any one of Claims 1 to 4, where the polyamide particles are present in concentrations ranging from 0.1 to 10% by weight and more particularly from 0.5 to 5% by weight relative to the total weight of the composition.

6. Composition according to any one of Claims 1 to 5, where the photoprotective system is constituted by one or more hydrophilic, lipophilic or insoluble organic screening agents and/or one or more inorganic screening agents.

7. Composition according to Claim 6, where the UV-screening agents are chosen from cinnamic derivatives; anthranilates; salicylic derivatives; dibenzoylmethane derivatives; camphor derivatives; benzophenone derivatives; β,β-diphenylacrylate derivatives; triazine derivatives; benzotriazole derivatives; benzalmalonate derivatives; benzimidazole derivatives; imidazolines; bisbenzoazolyl derivatives; p-aminobenzoic acid (PABA) derivatives; methylene bis(hydroxphenylbenzotriazole) derivatives; benzoxazole derivatives; screening polymers and screening silicones; α-alkylstyrene-derived dimers; 4,4-diarylbutadienes; merocyanine derivatives; and mixtures thereof.

8. Composition according to Claim 6, where the inorganic UV-screening agents of the invention are metal oxide particles having an average elementary particle size of less than or equal to 500 nm, more preferably between 5 nm and 500 nm and more preferably still between 10 nm and 100 nm.

9. Composition according to any one of Claims 1 to 8, **characterized in that** it contains at least one continuous aqueous phase and is in the form of a lotion or serum; in the form of an aqueous suspension; in the form of an aqueous gel; or in the form of a simple or complex (W/O/W) oil/water emulsion.

10. Composition according to Claim 9, in the form of an oil-in-water emulsion.

11. Use of the combination of at least one gelling starch and polyamide particles as defined in any of the preceding claims in a composition comprising, in a cosmetically acceptable support comprising at least one aqueous phase, at least one photoprotective system capable of screening out UV radiation, for the purpose of improving the cosmetic properties after application, said properties being chosen from the non-tacky effect, non-greasy effect, non-shiny effect and/or the absence of residual film.

## Patentansprüche

1. Zusammensetzung zum Schützen der Haut und/oder des Haars gegen Ultraviolettstrahlung, **dadurch gekennzeichnet, dass** sie mindestens:
(a) ein Lichtschutzsystem, das fähig ist, UV-Strahlung auszufiltern;
(b) mindestens eine gelierende Stärke, die nicht durch ein chemisches oder physikalisches Verfahren modifiziert ist und die aus unmodifizierten Maisstärken ausgewählt ist, und
(c) Polyamidpartikel
in einem mindestens eine wässrige Phase umfassenden kosmetisch unbedenklichen Träger umfasst.

2. Zusammensetzung nach Anspruch 1, wobei die gelierende Stärke derart ist, dass das Amylose-Amylopektin-Gewichtsverhältnis von 30/70 bis 16/84 schwanken kann.

3. Zusammensetzung nach einem der Ansprüche 1 oder 2, wobei die unmodifizierte(n) gelierende(n) Stärke(n) 0,1 bis 10 Gew.-%, vorzugweise 0,5 bis 5 Gew.-% und insbesondere 1 bis 3 Gew.-% in Bezug auf das Gesamtgewicht der Endzusammensetzung ausmachen kann/können.

4. Zusammensetzung nach einem der Ansprüche 1 bis 3, wobei die Polyamidpartikel Partikel von Polyamid PA-12 oder Polyamid PA-6 sind.

5. Zusammensetzung nach einem der Ansprüche 1 bis 4, wobei die Polyamidpartikel in Konzentrationen im Bereich von 0,1 bis 10 Gew.-%, insbesondere von 0,5 bis 5 Gew.-%, in Bezug auf das Gesamtgewicht der Zusammensetzung vorliegen.

6. Zusammensetzung nach einem der Ansprüche 1 bis 5, wobei das Lichtschutzsystem aus einem oder mehreren hydrophilen, lipophilen oder unlöslichen organischen Filtern und/oder einem oder mehreren anorganischen Filtern besteht.

7. Zusammensetzung nach Anspruch 6, wobei die UV-Filter aus Zimtsäurederivaten, Anthranilaten, Salicylsäurederivaten, Dibenzoylmethanderivaten, Kampferderivaten, Benzophenonderivaten, β,β-Diphenyl-acrylatderivaten, Triazinderivaten, Benzotriazolderivaten, Benzalmalonatderivaten, Benzimidazolderivaten, Imidazolinen, Bisbenzoazolylderivaten, p-Aminobenzoesäure(PABA)-Derivaten, Methylenbis(hydroxyphenylbenzotriazol)derivaten, Benzoxazolderivaten, Filterpolymeren und Filtersilikonen, von α-Alkylstyrol abgeleiteten Dimeren, 4,4-Diarylbutadienen, Merocyaninderivaten und Mischungen davon ausgewählt sind.

8. Zusammensetzung nach Anspruch 6, wobei die anorganischen UV-Filter der Erfindung Metalloxidpartikel mit einer durchschnittlichen Elementarpartikelgröße von 500 nm oder weniger, stärker bevorzugt zwischen 5 nm und 500 nm und noch stärker bevorzugt zwischen 10 nm und 100 nm aufweisen.

9. Zusammensetzung nach einem der Ansprüche 1 bis 8, **dadurch gekennzeichnet, dass** sie mindestens eine kontinuierliche wässrige Phase enthält und in Form einer Lotion oder eines Serums, in Form einer wässrigen Suspension, in Form eines wässrigen Gels oder in Form einer einfachen oder komplexen (W/O/W) Öl/Wasser-Emulsion vorliegt.

10. Zusammensetzung nach Anspruch 9 in Form einer Öl-in-Wasser-Emulsion.

11. Verwendung der Kombination von mindestens einer gelierenden Stärke und Polyamidpartikeln wie in einem der vorhergehenden Ansprüche definiert in einer Zusammensetzung, die mindestens eine wässrige Phase, mindestens ein Lichtschutzmittelsystem, das fähig ist, UV-Strahlung auszufiltern, in einem kosmetisch unbedenklichen Träger umfasst, zum Verbessern der kosmetischen Eigenschaften nach der Applikation, wobei die Eigenschaften aus frei von Klebrigkeit, frei von Fettigkeit, frei von Glanz und/oder Fehlen eines Rückstandsfilms ausgewählt sind.

## Revendications

1. Composition destinée à la protection de la peau et/ou des cheveux contre le rayonnement ultraviolet, **caractérisée par le fait qu'**elle comprend, dans un support cosmétiquement acceptable contenant au moins une phase aqueuse, au moins :
(a) un système photoprotecteur capable de filtrer le rayonnement UV ;
(b) au moins un amidon gélifiant non modifié par voie chimique ou physique, choisi parmi les amidons de maïs non modifiés, et
(c) des particules de polyamide.

2. Composition selon la revendication 1, où l'amidon gélifiant est tel que le rapport en poids amylose/amylopectine peut varier de 30/70 à 16/84.

3. Composition selon l'une quelconque des revendications 1 et 2, où le ou les amidons gélifiants non modifiés peuvent représenter de 0,1 à 10 % en poids, de préférence de 0,5 % à 5 % en poids et plus particulièrement de 1 à 3 % en poids par rapport au poids total de la composition finale.

4. Composition selon l'une quelconque des revendications 1 à 3, où les particules de polyamide sont des particules de polyamide PA-12 ou de polyamide PA-6.

5. Composition selon l'une quelconque des revendications 1 à 4, où les particules de polyamide sont présentes dans des concentrations allant de 0,1 à 10 % en poids et plus particulièrement de 0,5 à 5 % en poids par rapport au poids total de la composition.

6. Composition selon l'une quelconque des revendications 1 à 5, où le système photoprotecteur est constitué par un ou plusieurs filtres organiques hydrophiles, lipophiles ou insolubles et/ou un ou plusieurs filtres inorganiques.

7. Composition selon la revendication 6, où les filtres UV sont choisis parmi les dérivés cinnamiques ; les anthranilates ; les dérivés salicyliques ; les dérivés de dibenzoylméthane ; les dérivés du camphre ; les dérivés de la benzophénone ; les dérivés de β,β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes, les dérivés de mérocyanine et leurs mélanges.

8. Composition selon la revendication 6, où les filtres UV inorganiques de l'invention sont des particules d'oxyde métallique ayant une taille moyenne de particule élémentaire inférieure ou égale à 500 nm, plus préférentiellement comprise entre 5 nm et 500 nm et encore plus préférentiellement comprise entre 10 nm et 100 nm.

9. Composition selon l'une quelconque des revendications 1 à 8, caractérisée qu'elle contient au moins une phase aqueuse continue et se présente sous forme de lotion ou sérum ; sous forme de suspension aqueuse ; sous forme de gel aqueux ; ou sous forme d'émulsion huile/eau simple ou complexe (E/H/E).

10. Composition selon la revendication 9, sous forme d'émulsion huile-dans-eau.

11. Utilisation de l'association d'au moins un amidon gélifiant et de particules de polyamide telle que définie dans l'une quelconque des revendications précédentes dans une composition comprenant, dans un support cosmétiquement acceptable comprenant au moins une phase aqueuse, au moins un système photoprotecteur capable de filtrer le rayonnement UV, dans le but d'améliorer les propriétés cosmétiques après application, lesdites propriétés étant choisies parmi l'effet non-collant, l'effet non-gras, l'effet non-brillant et/ou l'absence de film résiduel.
